# EUROPÄISCHE PATENTSCHRIFT

(11) **EP 1 794 128 B1**
(45) Veröffentlichungstag und Bekanntmachung des Hinweises auf die Patenterteilung: **11.01.2012**
(21) Anmeldenummer: 05784511.7
(22) Anmeldetag: 08.09.2005
(51) Int. Cl.: C07D 217/22, A61K 31/47, A61P 9/12

(54) **SUBSTITUIERTE 4-PHENYLTETRAHYDROISOCHINOLINE, VERFAHREN ZU IHRER HERSTELLUNG, IHRE VERWENDUNG ALS MEDIKAMENT, SOWIE SIE ENTHALTENDES MEDIKAMENT**
SUBSTITUTED 4-PHENYLTETRAHYDROISOQUINOLINES, METHOD FOR THE PRODUCTION THEREOF, THEIR USE AS A MEDICAMENT, AND A MEDICAMENT CONTAINING THEM
4-PHENYLTETRAHYDRO-ISOQUINOLINES SUBSTITUEES, PROCEDE PERMETTANT DE LES PRODUIRE, LEUR UTILISATION COMME MEDICAMENT ET MEDICAMENT LES CONTENANT

(30) Priorität: 23.09.2004 DE 102004046492
(43) Veröffentlichungstag der Anmeldung: 13.06.2007
(73) Patentinhaber: Sanofi-Aventis Deutschland GmbH, 65929 Frankfurt am Main (DE)
(72) Erfinder: LANG, Hans-Jochen, 65719 Hofheim (DE); HEINELT, Uwe, 65926 Frankfurt am Main (DE); WIRTH, Klaus, 65830 Kriftel (DE); LICHER, Thomas, 65812 Bad Soden (DE); HOFMEISTER, Armin, 55278 Dexheim (DE)
(74) Vertreter: Michalski Hüttermann & Partner Patentanwälte
(86) Internationale Anmeldenummer: PCT/EP2005/009654
(87) Internationale Veröffentlichungsnummer: WO 2006/032372

(56) Entgegenhaltungen:
- WO-A-01/32624
- WO-A-03/048129
- WO-A-03/055880
- WO-A-2004/085404

## Beschreibung

Die Erfindung betrifft Verbindungen vom Typ der substituierten 4-Phenyltetrahydroisochinoline. Medikamente, die Verbindungen dieses Typs enthalten, sind nützlich bei der Prävention oder Behandlung diverser Erkrankungen. So lassen sich die Verbindungen unter anderem bei Nierenerkrankungen wie akutem oder chronischem Nierenversagen, bei Störungen der Gallenfunktion, bei Atemstörungen wie Schnarchen oder Schlafapnoen oder bei Schlaganfall einsetzen.

Die Erfindung betrifft Verbindungen der Formel I 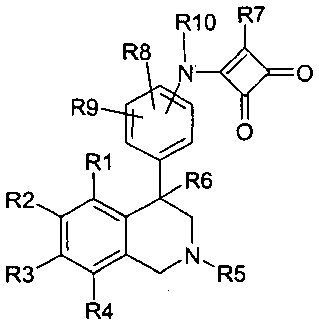 worin bedeuten:
- R1, R2, R3 und R4: unabhängig voneinander Wasserstoff, F, Cl, Br, I, CN, NO₂ oder R11-(CₘH₂₅ₘ)-Aₙ-;
m Null, 1, 2, 3 oder 4;
n Null oder 1;
R11 Wasserstoff, Methyl, CₚF₂ₚ₊₁ oder Phenyl;
p 1, 2 oder 3;
A Sauerstoff, NH, N(CH₃) oder S(O)q;
q Null, 1 oder 2;
- R5: Wasserstoff, Alkyl mit 1, 2, 3, 4, 5 oder 6 C-Atomen, das teilweise oder vollständig fluoriert sein kann, oder Cycloalkyl mit 3, 4, 5 oder 6 C-Atomen;
- R6: Wasserstoff, OH, F, CF₃, Methyl, Ethyl, Isopropyl oder Cyclopropyl;
- R7: Wasserstoff, Alkyl mit 1, 2, 3, 4, 5 oder 6 C-Atomen, Cycloalkyl mit 3, 4, 5 oder 6 C-Atomen, OR12 oder NR13R14;
R12 Wasserstoff, Alkyl mit 1, 2, 3, 4, 5 oder 6 C-Atomen, das teilweise oder vollständig fluoriert sein kann, oder Cycloalkyl mit 3, 4, 5 oder 6 C-Atomen;
R13 und R14 unabhängig voneinander Wasserstoff, Cycloalkyl mit 3, 4, 5 oder 6 C-Atomen, Alkyl mit 1, 2, 3, 4, 5 oder 6 C-Atomen, das teilweise oder vollständig fluoriert sein kann, Phenyl, Phenylalkyl mit 1, 2 oder 3 C-Atomen im Alkylteil, Heteroaryl oder Heteroarylmethyl, wobei Heteroarylreste aromatische Ringverbindungen sind, in denen ein oder mehrere Ringatome Sauerstoffatome, Schwefelatome oder Stickstoffatome sind, bevorzugt 1, 2, 3 oder 4 Stickstoffatome, 1 oder 2 Sauerstoffatome, 1 oder 2 Schwefelatome oder eine Kombinationen aus verschiedenen Heteroatomen und wobei weiterhin die Heteroarylreste über alle Positionen angebunden sein können, wobei die Phenyl- und Heteroarylreste unsubstituiert sind oder substituiert sind mit 1, 2, 3, 4 oder 5 Resten ausgewählt aus der Gruppe bestehend aus Fluor, Chlor, Methyl, CF₃, Methoxy und OH; und wobei die Alkylreste unsubstituiert sind oder substituiert sind mit 1, 2 oder 3 Resten ausgewählt aus der Gruppe Alkoxy mit 1, 2, 3 oder 4 C-Atomen, NR15R16 und Cycloalkyl mit 3, 4, 5 oder 6 C-Atomen;
R15 und R16unabhängig voneinander Wasserstoff oder Alkyl mit 1, 2, 3 oder 4 C-Atomen;
oder
R13 und R14 zusammen mit dem N-Atom, über das sie miteinander verbunden sind, einen 3, 4, 5, 6, 7, 8 oder 9-gliedrigen Ring, wobei ein C-Atom des Rings durch ein Sauerstoffatom oder eine NCH₃-Gruppe ersetzt sein kann,
- R8 und R9: unabhängig voneinander Wasserstoff, F, Cl, OH, CH₃, CH₃O, CF₃, CF₃CH₂O oder CH₃SO₂;
- R10: Wasserstoff, Methyl oder Ethyl; sowie deren pharmazeutisch verträgliche Salze und Trifluoracetate.

Bevorzugt sind Verbindungen der Formel I, worin bedeuten
- R1, R2, R3 und R4: unabhängig voneinander Wasserstoff, F, Cl, Br, CN oder R11-(CₘH₂ₘ)-Aₙ-;
m Null oder 1;
n Null oder 1;
R11 Wasserstoff, Methyl, CₚF₂ₚ₊₁- oder Phenyl;
p 1 oder 2;
A Sauerstoff oder S(O)q;
q Null, 1 oder 2;
- R5: Wasserstoff, Methyl, Ethyl oder Cyclopropyl;
- R6: Wasserstoff oder Methyl;
- R7: OR12 oder NR13R14;
R12 Wasserstoff oder Alkyl mit 1, 2 oder 3 C-Atomen;
R13 und R14 unabhängig voneinander Wasserstoff, Alkyl mit 1, 2, 3, 4, 5 oder 6 C-Atomen, das teilweise oder vollständig fluoriert sein kann, Cycloalkyl mit 3, 4, 5 oder 6 C-Atomen, Phenyl, Phenylalkyl mit 1, 2 oder 3 C-Atomen im Alkylteil, Heteroaryl oder Heteroarylmethyl, wobei Heteroarylreste aromatische Ringverbindungen sind, in denen ein oder mehrere Ringatome Sauerstoffatome, Schwefelatome oder Stickstoffatome sind, bevorzugt 1, 2, 3 oder 4 Stickstoffatome, 1 oder 2 Sauerstoffatome, 1 oder 2 Schwefelatome oder eine Kombinationen aus verschiedenen Heteroatomen und wobei weiterhin die Heteroarylreste über alle Positionen angebunden sein können,
wobei die Phenyl- und Heteroarylreste unsubstituiert sind oder substituiert sind mit 1, 2, 3, 4 oder 5 Resten ausgewählt aus der Gruppe bestehend aus Fluor, Chlor, Methyl, CF₃, Methoxy und OH;
wobei die Alkylreste unsubstituiert sind oder substituiert sind mit 1, 2 oder 3 Resten ausgewählt aus der Gruppe Alkoxy mit 1, 2, 3 oder 4 C-Atomen, NR15R16 und Cycloalkyl mit 3, 4, 5 oder 6 C-Atomen;
R15 und R16 nabhängig voneinander Wasserstoff oder Alkyl mit 1, 2, 3 oder 4 C-Atomen;
oder
R13 und R14 zusammen mit dem N-Atom, über das sie miteinander verbunden sind, einen 3, 4, 5, 6, 7 oder 8-gliedrigen Ring, wobei ein C-Atom des Rings durch ein Sauerstoffatom oder eine NCH₃-Gruppe ersetzt sein kann;
- R8 und R9: unabhängig voneinander Wasserstoff, F, Cl oder CH₃;
- R10: Wasserstoff oder Methyl;
sowie deren pharmazeutisch verträgliche Salze und Trifluoracetate.

Besonders bevorzugt sind Verbindungen der Formel I, worin bedeuten
- R1 und R3: Wasserstoff;
- R2 und R4: unabhängig voneinander Wasserstoff oder Cl;
- R5: Wasserstoff, Methyl oder Ethyl;
- R6: Wasserstoff oder Methyl;
- R7: OR12 oder NR13R14;
R12 Wasserstoff oder Alkyl mit 1, 2 oder 3 C-Atomen;
R13 und R14 unabhängig voneinander Wasserstoff, Alkyl mit 1, 2, 3, 4, 5 oder 6 C-Atomen, das teilweise oder vollständig fluoriert sein kann, Cycloalkyl mit 3, 4, 5 oder 6 C-Atomen, Phenyl, Phenylalkyl mit 1, 2 oder 3 C-Atomen im Alkylteil, Heteroaryl oder Heteroarylmethyl;, wobei Heteroarylreste aromatische Ringverbindungen sind, in denen ein oder mehrere Ringatome Sauerstoffatome, Schwefelatome oder Stickstoffatome sind, bevorzugt 1, 2, 3 oder 4 Stickstoffatome, 1 oder 2 Sauerstoffatome, 1 oder 2 Schwefelatome oder eine Kombinationen aus verschiedenen Heteroatomen und wobei weiterhin die Heteroarylreste über alle Positionen angebunden sein können,
wobei die Phenyl- und Heteroarylreste unsubstituiert sind oder substituiert sind mit 1, 2, 3, 4 oder 5 Resten ausgewählt aus der Gruppe bestehend aus Fluor, Chlor, Methyl, CF₃, Methoxy und OH;
wobei die Alkylreste unsubstituiert sind oder substituiert sind mit 1, 2 oder 3 Resten ausgewählt aus der Gruppe Alkoxy mit 1, 2, 3 oder 4 C-Atomen, NR15R16 und Cycloalkyl mit 3, 4, 5 oder 6 C-Atomen;
R15 und R16 unabhängig voneinander Wasserstoff oder Alkyl mit 1, 2, 3 oder 4 C-Atomen;
oder
R13 und R14 zusammen mit dem N-Atom, über das sie miteinander verbunden sind, einen 3, 4, 5, 6, 7 oder 8-gliedrigen Ring, wobei ein C-Atom des Rings durch ein Sauerstoffatom oder eine NCH₃-Gruppe ersetzt sein kann;
- R8 und R9: unabhängig voneinander Wasserstoff, F, Cl oder CH₃;
- R10: Wasserstoff oder Methyl;
sowie deren pharmazeutisch verträgliche Salze und Trifluoracetate.

Eine Ausführungsform betrifft Verbindungen der Formel I, in denen R1 Wasserstoff, F, Cl, Br, CN oder R11-(CₘH₂ₘ)-Aₙ- ist, wobei m Null oder 1 ist, n Null oder 1 ist, R11 Wasserstoff, Methyl, CₚF₂ₚ₊₁- oder Phenyl ist, wobei p 1 oder 2 ist, und A Sauerstoff oder S(O)q ist, wobei q Null, 1 oder 2 ist; bevorzugt sind Verbindungen der Formel I, in denen R1 Wasserstoff ist.

Eine weitere Ausführungsform betrifft Verbindungen der Formel I, in denen R2 Wasserstoff, F, Cl, Br, CN oder R11-(CₘH₂ₘ)-Aₙ- ist, wobei m Null oder 1 ist, n Null oder 1 ist, R11 Wasserstoff, Methyl, CₚF₂ₚ₊₁- oder Phenyl ist, wobei p 1 oder 2 ist, und A Sauerstoff oder S(O)q ist, wobei q Null, 1 oder 2 ist; bevorzugt sind Verbindungen der Formel I, in denen R2 Wasserstoff oder Cl, insbesondere Cl ist.

Eine weitere Ausführungsform betrifft Verbindungen der Formel I, in denen R3 Wasserstoff, F, Cl, Br, CN oder R11-(CₘH₂ₘ)-Aₙ- ist, wobei m Null oder 1 ist, n Null oder 1 ist, R11 Wasserstoff, Methyl, CₚF₂ₚ₊₁- oder Phenyl ist, wobei p 1 oder 2 ist, und A Sauerstoff oder S(O)q ist, wobei q Null, 1 oder 2 ist; bevorzugt sind Verbindungen der Formel I, in denen R3 Wasserstoff ist.

Eine weitere Ausführungsform betrifft Verbindungen der Formel I, in denen R4 Wasserstoff, F, Cl, Br, CN oder R11-(CₘH₂ₘ)-Aₙ- ist, wobei m Null oder 1 ist, n Null oder 1 ist, R11 Wasserstoff, Methyl, CₚF₂ₚ₊₁- oder Phenyl ist, wobei p 1 oder 2 ist, und A Sauerstoff oder S(O)q ist, wobei q Null, 1 oder 2 ist; bevorzugt sind Verbindungen der Formel I, in denen R4 Wasserstoff oder Cl, insbesondere Cl, ist.

Eine weitere Ausführungsform betrifft Verbindungen der Formel I, in denen R5 Wasserstoff, Methyl, Ethyl oder Cyclopropyl ist, bevorzugt Wasserstoff, Methyl oder Ethyl.

Eine weitere Ausführungsform betrifft Verbindungen der Formel I, in denen R6 Wasserstoff oder Methyl ist.

Eine weitere Ausführungsform betrifft Verbindungen der Formel I, in denen R7 OR12 oder NR13R14 ist, wobei R12 Wasserstoff oder Alkyl mit 1, 2 oder 3 C-Atomen ist und wobei R13 und R14 unabhängig voneinander Wasserstoff, Alkyl mit 1, 2, 3, 4, 5 oder 6 C-Atomen, das teilweise oder vollständig fluoriert sein kann, Cycloalkyl mit 3, 4, 5 oder 6 C-Atomen, Phenyl, Phenylalkyl mit 1, 2 oder 3 C-Atomen im Alkylteil, Heteroaryl oder Heteroarylmethyl sind, wobei Heteroarylreste aromatische Ringverbindungen sind, in denen ein oder mehrere Ringatome Sauerstoffatome, Schwefelatome oder Stickstoffatome sind, bevorzugt 1, 2, 3 oder 4 Stickstoffatome, 1 oder 2 Sauerstoffatome, 1 oder 2 Schwefelatome oder eine Kombinationen aus verschiedenen Heteroatomen und wobei weiterhin die Heteroarylreste über alle Positionen angebunden sein können wobei die Phenyl- und Heteroarylreste unsubstituiert oder substituiert sind mit 1, 2, 3, 4 oder 5 Resten ausgewählt aus der Gruppe bestehend aus Fluor, Chlor, Methyl, CF₃, Methoxy und OH und wobei wobei die Alkylreste unsubstituiert sind oder substituiert sind mit 1, 2 oder 3 Resten ausgewählt aus der Gruppe Alkoxy mit 1, 2, 3 oder 4 C-Atomen, insbesondere Methoxy, NR15R16, wobei R15 und R16 unabhängig voneinander Wasserstoff oder Alkyl mit 1, 2, 3 oder 4 C-Atomen, insbesondere Methyl, sind, und Cycloalkyl mit 3, 4, 5 oder 6 C-Atomen, insbesondere Cyclopropyl, oder wobei R13 und R14 zusammen mit dem N-Atom, über das sie miteinander verbunden sind, einen 3, 4, 5, 6, 7 oder 8-gliedrigen Ring bilden, wobei ein C-Atom des Rings durch ein Sauerstoffatom oder eine NCH₃-Gruppe ersetzt sein kann, beispielsweise bilden R13 und R14 zusammen mit dem N-Atom, über das sie miteinander verbunden sind, einen gesättigten Ring wie Pyrrolidino, Piperidino, Perhydroazepino, Morpholino, 4-Methylpiperazino, insbesondere Pyrrolidino.

Eine weitere Ausführungsform betrifft Verbindungen der Formel I, in denen R8 Wasserstoff, F, Cl oder Methyl ist.

Eine weitere Ausführungsform betrifft Verbindungen der Formel I, in denen R9 Wasserstoff, F, Cl oder Methyl ist.

Eine weitere Ausführungsform betrifft Verbindungen der Formel I, in denen R10 Wasserstoff oder Methyl ist.

Speziell bevorzugt sind Verbindungen der Formel I ausgewählt aus der Gruppe:
6,8-Dichlor-2-methyl-4-[4N-(2-ethoxy-3,4-dioxocyclobuten-1-yl)amino]phenyl-1,2,3,4-tetrahydroisochinolin,
6,8-Dichlor-2-methyl-4-[3N-(2-ethoxy-3,4-dioxocyclobuten-1-yl)amino]phenyl-1,2,3,4-tetrahydroisochinolin,
6,8-Dichlor-2-methyl-4-[2N-(2-ethoxy-3,4-dioxocyclobuten-1-yl)amino]phenyl-1,2,3,4-tetrahydroisochinolin,
6,8-Dichlor-2-methyl-4(S)-[4N-(2-ethoxy-3,4-dioxocyclobuten-1-yl)amino]phenyl-1,2,3,4-tetrahydroisochinolin,
6,8-Dichlor-2-methyl-4(S)-[3N-(2-ethoxy-3,4-dioxocyclobuten-1-yl)amino]phenyl-1,2,3,4-tetrahydroisochinolin,
6,8-Dichlor-2-methyl-4(R)-[2N-(2-ethoxy-3,4-dioxocyclobuten-1-yl)amino]phenyl-1,2,3,4-tetrahydroisochinolin,
6,8-Dichlor-2-methyl-4-[4N-(2-hydroxy-3,4-dioxocyclobuten-1-yl)amino]phenyl-1,2,3,4-tetrahydroisochinolin,
6,8-Dichlor-2-methyl-4-[3N-(2-hydroxy-3,4-dioxocyclobuten-1-yl)amino]phenyl-1,2,3,4-tetrahydroisochinolin,
6,8-Dichlor-2-methyl-4-[2N-(2-hydroxy-3,4-dioxocyclobuten-1-yl)amino]phenyl-1,2,3,4-tetrahydroisochinolin,
6,8-Dichlor-2-methyl-4-[4N-(2-amino-3,4-dioxocyclobuten-1-yl)amino]phenyl-1,2,3,4-tetrahydroisochinolin,
6,8-Dichlor-2-methyl-4-[3N-(2-amino-3,4-dioxocyclobuten-1-yl)amino]phenyl-1,2,3,4-tetrahydroisochinolin,
6,8-Dichlor-2-methyl-4-[2N-(2-amino-3,4-dioxocyclobuten-1-yl)amino]phenyl-1,2,3,4-tetrahydroisochinolin,
6,8-Dichlor-2-methyl-4(S)-[4N-(2-amino-3,4-dioxocyclobuten-1-yl)amino]phenyl-1,2,3,4-tetrahydroisochinolin,
6,8-Dichlor-2-methyl-4(S)-[3N-(2-amino-3,4-dioxocyclobuten-1-yl)amino]phenyl-1,2,3,4-tetrahydroisochinolin,
6,8-Dichlor-2-methyl-4(R)-[2N-(2-amino-3,4-dioxocyclobuten-1-yl)amino]phenyl-1,2,3,4-tetrahydroisochinolin,
6,8-Dichlor-2-methyl-4-[4N-(2-N-cyclohexyl-N-methylamino-3,4-dioxocyclobuten-1-yl)amino]phenyl-1,2,3,4-tetrahydroisochinolin,
6,8-Dichlor-2-methyl-4-[4N-(2-dimethylamino-3,4-dioxocyclobuten-1-yl)amino]phenyl-1,2,3,4-tetrahydroisochinolin,
6,8-Dichlor-2-methyl-4-[3N-(2-dimethylamino-3,4-dioxocyclobuten-1-yl)amino]phenyl-1,2,3,4-tetrahydroisochinolin,
6,8-Dichlor-2-methyl-4(S)-[4N-(2-dimethylamino-3,4-dioxocyclobuten-1-yl)amino]phenyl-1,2,3,4-tetrahydroisochinolin,
6,8-Dichlor-2-methyl-4(R)-[2N-(2-dimethylamino-3,4-dioxocyclobuten-1-yl)amino]phenyl-1,2,3,4-tetrahydroisochinolin,
6,8-Dichlor-2-methyl-4(S)-[3N-(2-dimethylamino-3,4-dioxocyclobuten-1-yl)amino]phenyl-1,2,3,4-tetrahydroisochinolin,
6,8-Dichlor-2-methyl-4(S)-[4N-(2-N-(1-butyl)-N-methylamino-3,4-dioxocyclobuten-1-yl)amino]phenyl-1,2,3,4-tetrahydroisochinolin,
6,8-Dichlor-2-methyl-4-[4N-(2-dipropylamino-3,4-dioxocyclobuten-1-yl)amino]phenyl-1,2,3,4-tetrahydroisochinolin,
6,8-Dichlor-2-methyl-4-[4N-(2-(N-isobutyl-N-methylamino)-3,4-dioxocyclobuten-1-yl)amino]phenyl-1,2,3,4-tetrahydroisochinolin,
6,8-Dichlor-2-methyl-4-[4N-(2-(N-methoxyethyl)-N-methylamino)-3,4-dioxocyclobuten-1-yl)amino]phenyl-1,2,3,4-tetrahydroisochinolin,
6,8-Dichlor-2-methyl-4(S)-[4N-(2-diethylamino-3,4-dioxocyclobuten-1-yl)amino]phenyl-1,2,3,4-tetrahydroisochinolin,
6,8-Dichlor-2-methyl-4(S)-[4N-(2-(N-isopropyl-N-methylamino)-3,4-dioxo-cyclobuten-1-yl)amino]phenyl-1,2,3,4-tetrahydroisochinolin,
6,8-Dichlor-2-methyl-4-[4N-(2-(N-cyclopropylmethyl-N-propylamino)-3,4-dioxocyclobuten-1-yl)amino]phenyl-1,2,3,4-tetrahydroisochinolin,
6,8-Dichlor-2-methyl-4(S)-[3N-(2-diethylamino-3,4-dioxocyclobuten-1-yl)amino]phenyl-1,2,3,4-tetrahydroisochinolin,
6,8-Dichlor-2-methyl-4(S)-[3N-(2-N-methyl-N-isopropyl)amino-3,4-dioxocyclobuten-1-yl)amino]phenyl-1,2,3,4-tetrahydroisochinolin,
6,8-Dichlor-2-methyl-4(S)-[4N-(2N-ethyl-N-isopropylamino)-3,4-dioxocyclobuten-1-yl)amino]phenyl-1,2,3,4-tetrahydroisochinolin,
6,8-Dichlor-2-methyl-4(S)-[3N-(2-N-methyl-N-propylamino)-3,4-dioxocyclobuten-1-yl)amino]phenyl-1,2,3,4-tetrahydroisochinolin,
6,8-Dichlor-2-methyl-4(S)-[3N-(2-N-pyrrolidino-3,4-dioxocyclobuten-1-yl)amino]phenyl-1,2,3,4-tetrahydroisochinolin,
6,8-Dichlor-2-methyl-4(S)-[4N-(2-N-cyclopropylamino)-3,4-dioxo-cyclobuten-1-yl)amino]phenyl-1,2,3,4-tetrahydroisochinolin,
6,8-Dichlor-2-methyl-4(S)-[4N-(2-N-pyrrolidino-3,4-dioxocyclobuten-1-yl)amino]phenyl-1,2,3,4-tetrahydroisochinolin,
6,8-Dichlor-2-methyl-4(R)-[2N-(2-N-methyl-N-propylamino)-3,4-dioxocyclobuten-1-yl)amino]phenyl-1,2,3,4-tetrahydroisochinolin,
6,8-Dichlor-2-methyl-4-[4N-(2-N-benzyl-N-methylamino)-3,4-dioxo-cyclobuten-1-yl)amino]phenyl-1,2,3,4-tetrahydroisochinolin,
6,8-Dichlor-2-methyl-4(R)-[2N-(2-diethylamino-3,4-dioxocyclobuten-1-yl)amino]phenyl-1,2,3,4-tetrahydroisochinolin,
6,8-Dichlor-2-methyl-4(R)-[2N-(2-dipropylamino)-3,4-dioxocyclobuten-1-yl)amino]phenyl-1,2,3,4-tetrahydroisochinolin,
6,8-Dichlor-2-methyl-4(R)-[2N-(2-N-pyrrolidino-3,4-dioxocyclobuten-1-yl)amino]phenyl-1,2,3,4-tetrahydroisochinolin,
6,8-Dichlor-2-methyl-4(R)-[2N-(2-N-methyl-N-isopropyl)amino-3,4-dioxocyclobuten-1-yl)amino]phenyl-1,2,3,4-tetrahydroisochinolin,
6,8-Dichlor-2-methyl-4-[4N-(2-N-phenylamino-3,4-dioxocyclobuten-1-yl)amino]phenyl-1,2,3,4-tetrahydroisochinolin,
6,8-Dichlor-2-methyl-4-[2N-(2-N-methylamino-3,4-dioxocyclobuten-1-yl)amino]phenyl-1,2,3,4-tetrahydroisochinolin,
6,8-Dichlor-2-methyl-4-[2N-(2-N-(1-hexylamino)-3,4-dioxocyclobuten-1-yl)amino]phenyl-1,2,3,4-tetrahydroisochinolin,
6,8-Dichlor-2-methyl-4-[2N-(2-N-isopropylamino-3,4-dioxocyclobuten-1-yl)amino]phenyl-1,2,3,4-tetrahydroisochinolin,
6,8-Dichlor-2-methyl-4-[2N-(2-N-cyclopentylamino-3,4-dioxocyclobuten-1-yl)amino]phenyl-1,2,3,4-tetrahydroisochinolin,
6,8-Dichlor-2-methyl-4-[2N-(2N-(2-furylmethyl)amino-3,4-dioxocyclobuten-1-yl)amino]phenyl-1,2,3,4-tetrahydroisochinolin,
6,8-Dichlor-2-methyl-4-[2N-(2-N-ethylamino-3,4-dioxocyclobuten-1-yl)amino]phenyl-1,2,3,4-tetrahydroisochinolin,
6,8-Dichlor-2-methyl-4-[2N-(2-N-dimethylaminoethylamino-3,4-dioxocyclobuten-1-yl)amino]phenyl-1,2,3,4-tetrahydroisochinolin,
6,8-Dichlor-2-methyl-4-[4N-(2-N-ethylamino-3,4-dioxocyclobuten-1-yl)amino]phenyl-1,2,3,4-tetrahydroisochinolin,
6,8-Dichlor-2-methyl-4-[2N-(2N-(3-picolylmethyl)amino-3,4-dioxocyclobuten-1-yl)amino]phenyl-1,2,3,4-tetrahydroisochinolin,
6,8-Dichlor-2-methyl-4-[4N-(2N-methylamino-3,4-dioxocyclobuten-1-yl)amino]phenyl-1,2,3,4-tetrahydroisochinolin,
6,8-Dichlor-2-methyl-4-[4N-(2N-cyclopropylamino-3,4-dioxocyclobuten-1-yl)amino]phenyl-1,2,3,4-tetrahydroisochinolin,
6,8-Dichlor-2-methyl-4-[4N-(2N-isopropylamino-3,4-dioxocyclobuten-1-yl)amino]phenyl-1,2,3,4-tetrahydroisochinolin,
6,8-Dichlor-2-methyl-4-[4N-(2N-(2-furylmethyl)amino-3,4-dioxocyclobuten-1-yl)amino]phenyl-1,2,3,4-tetrahydroisochinolin,
6,8-Dichlor-2-methyl-4-[4N-(2N-dimethylaminoethylamino)-3,4-dioxocyclobuten-1-yl)amino]phenyl-1,2,3,4-tetrahydroisochinolin,
6,8-Dichlor-2-methyl-4-[4N-(1-hexylamino)-3,4-dioxocyclobuten-1-yl)amino]phenyl-1,2,3,4-tetrahydroisochinolin,
6,8-Dichlor-2-methyl-4-[4N-(3-picolylamino)-3,4-dioxo-cyclobuten-1-yl)amino]phenyl-1,2,3,4-tetrahydroisochinolin,
6,8-Dichlor-2-methyl-4-[4N-2-methylamino-3,4-dioxo-cyclobuten-1-yl)amino]phenyl-1,2,3,4-tetrahydroisochinolin,
6,8-Dichlor-2-methyl-4-[4N-(2-ethylamino)-3,4-dioxo-cyclobuten-1-yl)amino]phenyl-1,2,3,4-tetrahydroisochinolin,
6,8-Dichlor-2-methyl-4-[3N-(2-(2-furylmethylamino)-3,4-dioxo-cyclobuten-1-yl)amino]phenyl-1,2,3,4-tetrahydroisochinolin,
6,8-Dichlor-2-methyl-4-[3N-(2-cyclopentylamino-3,4-dioxo-cyclobuten-1-yl)amino]phenyl-1,2,3,4-tetrahydroisochinolin,
6,8-Dichlor-2-methyl-4-[3N-(2-isopropylamino)-3,4-dioxo-cyclobuten-1-yl)amino]phenyl-1,2,3,4-tetrahydroisochinolin,
6,8-Dichlor-2-methyl-4-[3N-(2N-dimethylaminoethylamino)-3,4-dioxocyclobuten-1-yl)amino]phenyl-1,2,3,4-tetrahydroisochinolin,
6,8-Dichlor-2-methyl-4(R)-[2N-(2-isopropylamino-3,4-dioxocyclobuten-1-yl)amino]phenyl-1,2,3,4-tetrahydroisochinolin,
6,8-Dichlor-2-methyl-4(R)-[3N-(2-methylamino-3,4-dioxocyclobuten-1-yl)amino]phenyl-1,2,3,4-tetrahydroisochinolin,
   und
6,8-Dichlor-2-methyl-4(R)-[2N-(2-methylamino-3,4-dioxocyclobuten-1-yl)amino]phenyl-1,2,3,4-tetrahydroisochinolin
sowie deren pharmazeutisch verträgliche Salze und Trifluoracetate.

Enthalten die Verbindungen der Formel I ein oder mehrere Asymmetriezentren, so können diese unabhängig voneinander sowohl S als auch R konfiguriert sein. Die Verbindungen können als optische Isomere, als Enantiomere, als Diastereomere, als Racemate oder als Gemische in allen Verhältnissen derselben vorliegen.

Die vorliegende Erfindung umfasst alle tautomeren Formen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl oder Hexyl. Bevorzugte Alkylreste sind Methyl, Ethyl, n-Propyl, Isopropyl, n-Butyl, tert-Butyl, n-Pentyl, n-Hexyl. In Alkylresten können ein oder mehrere, zum Beispiel 1, 2, 3, 4, 5, 6, 7, 8, 9, 10, 11, 12 oder 13, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl.

Beispiele für Cycloalkylreste sind Cyclopropyl, Cyclobutyl, Cyclopentyl, Cyclohexyl, Cycloheptyl oder Cyclooctyl. In den Cycloalkylresten können eine oder mehrere CH₂-Gruppen durch O, NH oder N-Alkyl, beispielsweise NCH₃, ersetzt sein.

Phenylreste können unsubstituiert sein oder einfach oder mehrfach, zum Beispiel einfach, zweifach oder dreifach, durch gleiche oder verschiedene Reste substituiert sein. Dies gilt ebenso für substituierte Phenylreste in Gruppen wie Phenylalkyl. Phenylalkylreste sind zum Beispiel Benzyl, 1-Phenylethyl oder 2-Phenylethyl. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Heteroarylreste sind aromatische Ringverbindungen, in denen ein oder mehrere Ringatome Sauerstoffatome, Schwefelatome oder Stickstoffatome sind, z. B. 1, 2, 3 oder 4 Stickstoffatome, 1 oder 2 Sauerstoffatome, 1 oder 2 Schwefelatome oder eine Kombinationen aus verschiedenen Heteroatomen. Die Heteroarylreste können über alle Positionen angebunden sein, zum Beispiel über 1-Position, 2-Position, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein. Dies gilt ebenso für substituierte Heteroarylreste in Gruppen wie Heteroarylmethyl. Als Heteroaryl gelten beispielsweise 2- oder 3-Thienyl, 2- oder 3-Furyl, 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also zum Beispiel 1-Oxy-2-, -3- oder -4-pyridyl. Bevorzugt sind dabei die 5- oder 6-gliedrigen Heterocyclen, zum Beispiel Imidazolyl, Pyrazolyl, Pyrrolyl, Triazolyl, Tetrazolyl, Thiazolyl, Thienyl, Furyl, Oxazolyl und Pyridyl.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I an einer sauren Gruppe deprotoniert werden und beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren, verwendet werden. Verbindungen der Formel I, die eine basische Gruppe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw.

Gegenstand der Erfindung ist auch das im folgenden beschriebene Verfahren zur Herstellung der Verbindungen der Formel I.

Die hier beschriebenen Verbindungen der Formel I lassen sich ausgehend von den Anilin-Derivaten der Formel II herstellen. Hierzu werden Verbindungen der Formel II mit Quadratsäurederivaten der Formel III umgesetzt, 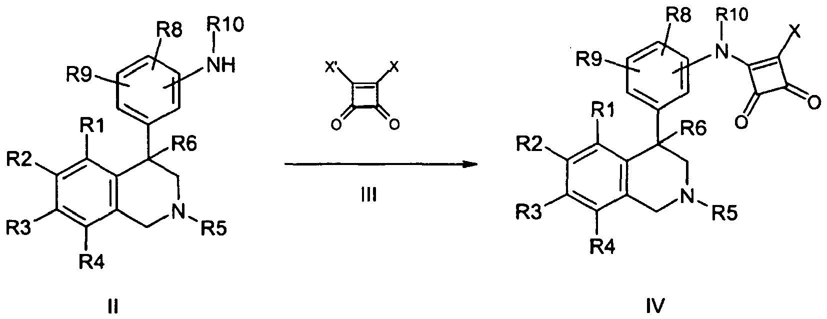 wobei die Substituenten R1, R2, R3, R4, R5, R6, R8, R9 und R10 wie oben beschrieben definiert sind, X eine nucleophil gut substituierbare Gruppe wie Chlor oder Phenoxy ist oder wie R7 definiert ist und X' eine nucleophil substituierbare Gruppe ist, zum Beispiel Chlor, Phenoxy oder Alkoxy, beispielsweise Ethoxy.
Die Verbindungen der Formel IV entsprechen Verbindungen der Formel I, wenn der Substituent X die Bedeutung von R7 besitzt.
Die Quadratsäurederivate der Formel III sind käuflich erhältlich oder nach literaturbekannten Verfahren herstellbar.

Weiterhin können die Verbindungen der Formel IV in vielfältiger Weise durch nucleophilen Austausch mit Verbindungen der Formel V durch dem Fachmann bekannte Verfahren in erfindungsgemäßen Verbindungen der Formel I übergeführt werden, 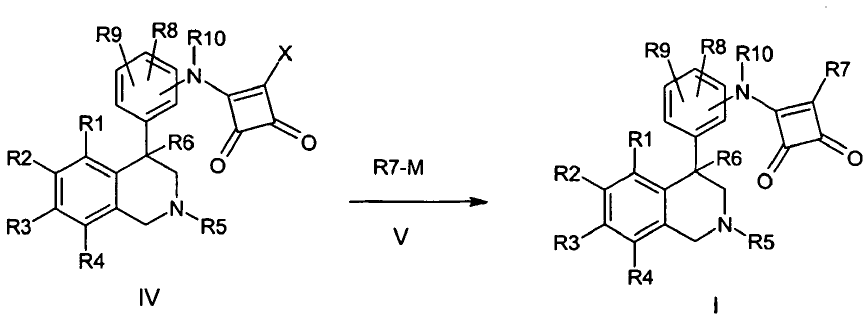 wobei R1, R2, R3, R4, R5, R6, R7, R8, R9, R10 und die oben angegebene Bedeutung besitzen, X eine nucleophil substituierbare Gruppe ist, zum Beispiel Chlor, Phenoxy oder Alkoxy, beispielsweise Ethoxy, und M entweder Wasserstoff oder ein Metall, insbesondere ein Alkalimetall oder ein Erdalkalimetalläquivalent bedeutet, beispielsweise Lithium oder Gringardverbindungen.
Die Verbindungen der Formel V sind käuflich erhältlich oder können nach literaturbekannten Verfahren hergestellt werden.

Die Darstellung von Tetrahydroisochinolinen der Formel II, in denen R6 Wasserstoff bedeutet (Tetrahydroisochinoline der Formel IIa), kann beispielsweise durch Reduktion der Carbonylgruppierung in Verbindungen der Formel VI und anschließende säurekatalysierte Cyclisierung der entsprechenden Alkohole der Formel VII (vgl. Tetrahedron Lett., 1989, 30, 5837; Org. Prep. Proced. Int., 1995, 27, 513) nach bekannten Verfahren erfolgen, 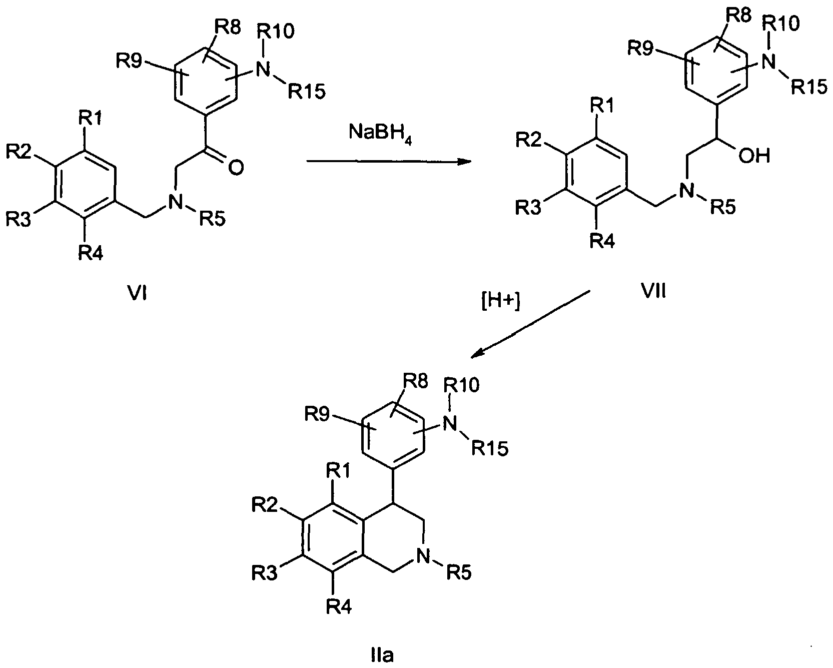 wobei die Substituenten R1, R2, R3, R4, R5, R8, R9 und R10 die oben angegebene Bedeutung besitzen und R15 Wasserstoff oder eine dem Fachmann geläufige Stickstoff-Schutzgruppe bedeutet, beispielsweise ein Acetylrest.

Die oben verwendeten Verbindungen der Formel VI können in dem Fachmann bekannter Weise durch Alkylierung der Benzylamine der Formel VIII mit den entsprechend substituierten alpha-Bromacetophenonverbindungen IX hergestellt werden, 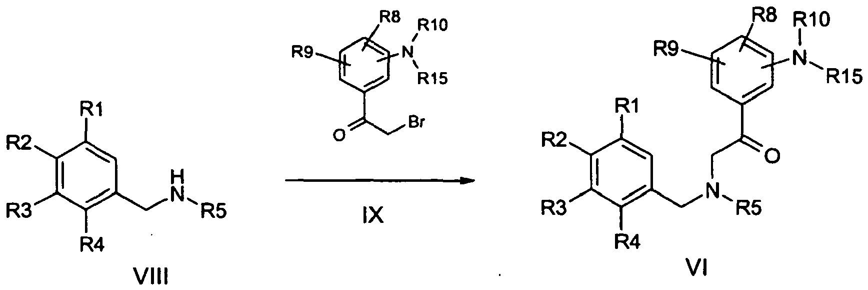 wobei die Substituenten R1, R2, R3, R4, R5, R8, R9, R10 und R15 die oben angegebene Bedeutung haben.
Die alpha-Bromacetophenonverbindungen der Formel IX lassen sich in literaturbekannten Verfahren aus den entsprechenden Acetophenonvorläufern durch Bromierung gewinnen, wie beispielsweise beschrieben in Jerry March, Advanced Organic Chemistry, Reactions, Mechanisms, and Structure. Second Edition, 7th Printing,McGraw-Hill Book Company Japan, Ltd 1983, pages 537-539, Chapter: "Halogenation of Aldehydes and Ketones.

Die Benzylamin-Vorläufer der Formel VIII können, falls nicht käuflich erhältlich, nach dem Fachmann bekannten Standardverfahren beispielsweise aus den entsprechenden Benzylchloriden oder -bromiden der Formel X und dem entsprechenden Amin synthetisiert werden, 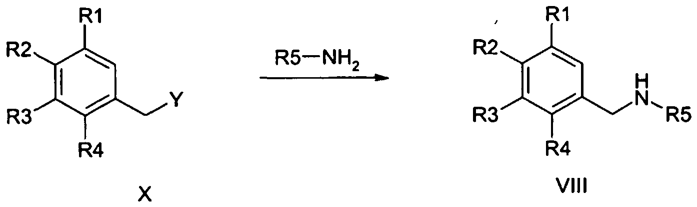 wobei R1, R2, R3, R4 und R5 wie oben definiert sind und Y Halogen, beispielsweise Cl oder Br ist.

Zur Herstellung verzweigter Verbindungen der Formel II, in denen R6 nicht Wasserstoff bedeutet (Verbindungen der Formel IIb), können beispielsweise die entsprechenden Diphenylessigsäureester der Formel XI in alpha-Stellung mit R6 nach bekannten Methoden alkyliert werden. Die erhaltenen Verbindungen der Formel XII können durch Standardverfahren in die entsprechenden Amide der Formel XIII überführt werden, welche in einer Pictet-Spengler-analogen Reaktion in die gewünschten Tetrahydroisochinoline IIb überführt werden können (vgl. Tetrahedron; 1987, 43, 439; Chem. Pharm. Bull.; 1985, 33, 340), 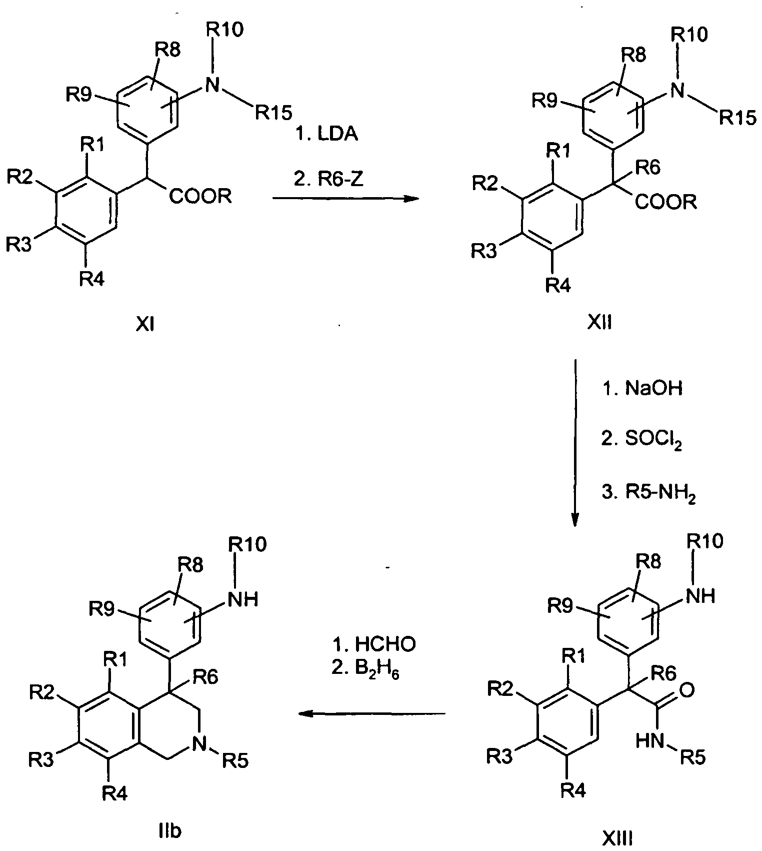 wobei die Substituenten R1, R2, R3, R4, R5, R6, R7, R8, R9, R10 und R15 die oben angegebenen Bedeutungen besitzen und wobei Z ein Halogen ist, beispielsweise Cl, Br oder I, und R ein Alkyl mit 1, 2, 3 oder 4 C-Atomen, beispielsweise Methyl oder Ethyl, ist.
Die Verbindungen R5-NH₂ und R6-Z sind käuflich erhältlich oder nach literaturbekannten Verfahren herstellbar.

Die Verbindungen der Formel XI können, wenn sie nicht käuflich erhältlich sind, beispielsweise aus einem entsprechenden Benzylcyanid erhalten werden, welches in alpha-Stellung unter basischer Zweiphasenkatalyse mit einem Nitro-substituierten Fluor- oder Chlorbenzol phenyliert wird; das resultierende alpha-Phenyl-Benzylcyanid kann basisch oder bevorzugt im sauren Medium zur entsprechenden Carbonsäure XI hydrolysiert werden.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Es konnte gezeigt werden, dass Verbindungen der Formel I hervorragende Inhibitoren des Natrium-Wasserstoffaustauschers (NHE), insbesondere des Natrium-Wasserstoffaustauschers vom Subtyp 3 (NHE3), darstellen.

Die bisher bekannten NHE3-Inhibitoren leiten sich beispielsweise von Verbindungen des Acylguanidin-Typs (EP825178), Norbornylamin-Typs (WO0144164), 2-Guanidinochinazolin-Typs (WO0179186) oder Benzamidin-Typs (WO0121582, WO0172742) ab. Das ebenfalls als NHE3-Inhibitor beschriebene Squalamin (M. Donowitz et al. Am. J. Physiol. 276 (Cell Physiol. 45): C136 - C144) wirkt nach derzeitigem Wissensstand nicht unmittelbar wie die Verbindungen nach Formel I, sondern über einen indirekten Mechanismus und erreicht so seine maximale Wirkstärke erst nach einer Stunde. Solche mechanistisch andersartig wirkende NHE3-Inhibitoren eignen sich beispielsweise als Kombinationspartner der vorliegenden erfindungsgemäßen Verbindungen.

Tetrahydroisochinoline als Inhibitoren des Natrium-Wasserstoffaustauschers vom Subtyp 3 (NHE3) werden bereits in den Anmeldungen WO03048129 und DE10312963 beschrieben. In der Patentanmeldung WO03055880 ist die verwandte Verbindungsklasse der Tetrahydroisochinolinium-Salze als NHE3-Inhibitoren beschrieben. Überraschend wurde nun gefunden, dass die hier beschriebenen Verbindungen der Formel I ebenfalls potente Inhibitoren des NHE3 darstellen und dabei vorteilhafte pharmakologische und pharmakokinetische Eigenschaften besitzen. So zeichnen sich die Verbindungen durch verbesserte Eigenschaften, wie beispielsweise eine hohe Selektivität gegenüber dem Natrim-Wasserstoff-Austauscher ohne nennenswerte Wirkung auf hERG-Kaliumkanal, aus.

Der NHE3 wird im Körper verschiedener Spezies bevorzugt in der Galle, dem Darm und in der Niere gefunden (Larry Fliegel et al., Biochem. Cell. Biol. 76: 735 - 741, 1998), ließ sich aber auch im Gehirn nachweisen (E. Ma et al., Neuroscience 79: 591 - 603).

Aufgrund der NHE-inhibitorischen Eigenschaften eignen sich die Verbindungen der Formel I und deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.

Generell können die hier beschriebenen NHE Inhibitoren in günstiger Weise mit anderen, den intrazellulären pH-Wert ebenfalls regulierenden Verbindungen kombiniert werden, wobei Inhibitoren der Enzymgruppe der Carboanhydratasen, Inhibitoren der Bicarbonationen transportierenden Systeme wie des Natrium-Bicarbonat-Cotransporters (NBC) oder des Natrium-abhängigen Chlorid-Bicarbonat-Austauschers (NCBE), sowie mit anderen NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

Die pharmakologische Wirkung der Verbindungen der Formel I ist dadurch gekennzeichnet, dass sie zu einer Verbesserung des Atemantriebes führen. Sie können deshalb zur Behandlung von gestörten Atmungszuständen herangezogen werden, wie sie beispielsweise bei folgenden klinischen Zuständen und Krankheiten auftreten können: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird. Die genannten Verbindungen finden deshalb vorteilhaft Verwendung zur Herstellung eines Medikaments zur Prävention und Behandlung von Schlafapnoen und muskulär bedingter Atemstörungen und zur Herstellung eines Medikaments zur Prävention und Behandlung des Schnarchens.

Eine Kombination eines NHE-Inhibitors der Formel I mit einem Carboanhydrase-Hemmer (z. B. Acetazolamid) kann sich als vorteilhaft erweisen, wobei letzterer eine metabolische Azidose herbeiführt und dadurch bereits die Atmungstätigkeit steigert, so dass eine verstärkte Wirkung und verminderter Wirkstoffeinsatz erzielt werden können.

Die erfindungsgemäßen Verbindungen schonen infolge ihrer NHE3-inhibitorischen Wirkung die zellulären Energiereserven, die sich unter toxischen und pathogenen Ereignissen rasch erschöpfen und so zur Zellschädigung oder zum Zelluntergang führen. Dabei wird die energieaufwendige ATP-verbrauchende Natriumresorption im proximalen Tubulus unter dem Einfluß von NHE3-Inhibitoren vorübergehend still gelegt und die Zelle kann so eine akute pathogene, ischämische oder toxische Situation überleben. Die Verbindungen eignen sich deshalb beispielsweise als Arzneimittel zur Behandlung ischämischer Noxen, zum Beispiel des akuten Nierenversagens. Weiterhin eignen sich die Verbindungen auch zur Behandlung aller chronischen Nierenerkrankungen und Nephritisformen, die infolge vermehrter Proteinausscheidung zum chronischen Nierenversagen führen. Dementsprechend eignen sich die Verbindungen der Formel I zur Herstellung eines Medikaments zur Behandlung diabetischer Spätschäden, der diabetischen Nephropathie und chronischer Nierenerkrankungen, insbesondere von allen Nierenentzündungen (Nephritiden), die mit einer vermehrten Protein-/ Albuminausscheidung verbunden sind.

Es hat sich gezeigt, dass die erfindungsgemäß verwendeten Verbindungen eine milde abführende Wirkung besitzen und demzufolge auch vorteilhaft als Abführmittel oder bei drohender Darmverstopfung verwendet werden können.

Weiterhin können die erfindungsgemäßen Verbindungen vorteilhaft zur Prävention und Therapie von akuten und chronischen Erkrankungen des Darmtrakts verwendet werden, die beispielweise durch ischämische Zustände im Intestinalbereich und / oder durch nachfolgende Reperfusion oder durch entzündliche Zustände und Ereignisse ausgelöst werden. Solche Komplikationen können beispielweise durch mangelnde Darmperistaltik auftreten, wie sie z.B. häufig nach chirurgischen Eingriffen, bei Darmverstopfung oder stark verminderter Darmtätigkeit zu beobachten sind.

Mit den erfindungsgemäßen Verbindungen besteht die Möglichkeit, der Gallenstein-Bildung vorzubeugen.

Die erfindungsgemäßen NHE-Inhibitoren eignen sich generell zur Behandlung von Krankheiten, die durch Ischämie und durch Reperfusion hervorgerufen werden.

Die erfindungsgemäßen Verbindungen sind infolge ihrer pharmakologischen Eigenschaften als antiarrhythmische Arzneimittel geeignet.
Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die erfindungsgemäßen Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den mit Verbindungen der Formel I vorbehandelten Empfängerorganismus verwendet werden können.

Die Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind.

Da NHE-Inhibitoren menschliches Gewebe und Organe nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist deren kombinierte Verabreichung mit Verbindungen der Formel I geeignet, die cytotoxischen Effekte einer Therapie zu vermindern oder zu unterdrücken. Durch die Verminderung der cytotoxischen Effekte, insbesondere der Cardiotoxizität, infolge Ko-Medikation mit NHE-Inhibitoren kann außerdem die Dosis der cytotoxischen Therapeutika erhöht und / oder die Medikation mit solchen Arzneimitteln verlängert werden. Der therapeutische Nutzen einer solchen cytotoxischen Therapie kann durch die Kombination mit NHE-Inhibitoren erheblich gesteigert werden.
Die Verbindungen der Formel I eignen sich insbesondere zur Verbesserung der Therapie mit Arzneimitteln, die eine unerwünschte cardiotoxische Komponente besitzen.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind. Die Verbindungen der Formel I eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychischer Depressionszustände, Angsterkrankungen und Psychosen. Dabei können die erfindungsgemäßen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Die Verbindungen der Formel I können ebenfalls zur Prävention und zur Behandlung thrombotischer Erkrankungen verwendet werden, da sie als NHE-Inhibitoren sowohl die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und thrombogener Selectin-Proteine, hemmen bzw. verhindern. Damit kann die pathogene Wirkung thrombogener und entzündungsrelevanter Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-RezeptorAntagonisten, Phosphodiesterase-Inhibitoren, Faktor-VIIa-Antagonisten, Clopidogrel, Ticlopidin usw. Eine kombinierte Anwendung der vorliegenden NHE-Inhibitoren mit - NCBE-Inhibitoren und/oder mit Inhibitoren der Carboanhydratase, wie beispielsweise mit Acetazolamid, ist besonders günstig.

Darüber hinaus zeichnen sich die erfindungsgemäßen NHE-Inhibitoren durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden. Sie können somit zur Behandlung von Organhypertrophien und -hyperplasien, beispielsweise des Herzens und der Prostata verwendet werden. Verbindungen der Formel I sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

NHE-Inhibitoren zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.

Da der NHE bei essentiellen Hypertonikern signifikant erhöht ist, eignen sich die Verbindungen der Formel I zur Prävention und zur Behandlung des Bluthochdrucks und von Herz-Kreislauferkrankungen. Dabei können sie allein oder mit einem geeigneten Kombinationspartner zur Bluthochdruckbehandlung und zur Behandlung von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, mit Verbindungen der Formel I kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch ß-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromokalim, Minoxidil und deren Derivate, Aktivatoren des mitochondrialen ATP-sensitiven Kaliumkanals (mitoK(ATP) Kanal), Inhibitoren weiterer Kaliumkanäle, wie des Kv1.5 usw.

Infolge ihrer antiphlogistischen Wirkung können erfindungsgemäße NHE-Inhibitoren als Antünflammatorika verwendet werden. Mechanistisch fällt dabei die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antünflammatorika verwendet.

Es wurde außerdem gefunden, dass NHE-Inhibitoren eine günstige Beeinflussung der Serumlipoproteine zeigen. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades. NHE-Inhibitoren der Formel I finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und zur Behandlung kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, stellt eine günstige Kombination mit verstärkter Wirkung und vermindertem Wirkstoffeinsatz dar.

So führen NHE-Inhibitoren zu einen wirksamen Schutz gegen Endothelschädigungen unterschiedlicher Genese. Mit diesem Schutz der Gefäße gegen das Syndrom der endothelialen Dysfunktion sind NHE-Inhibitoren wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem eignen sich NHE-Inhibitoren zur Behandlung des nicht-insulinabhängigen Diabetes (NIDDM), wobei beispielweise die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR-Agonisten, wie Rosiglitazone, Pioglitazone etc, mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken NHE-Inhibitoren der Entstehung diabetischer Spätkomplikation entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den oben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

NHE-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die auch unabhängig von akuten Mangeldurchblutungszuständen sind und auch bei normalen, nicht-ischämischen Bedingungen auftreten können. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. NHE-Inhibitoren eignen sich somit hervorragend zur Behandlung und Prävention der altersbedingt fortschreitenden endothelialen Dysfunktion, insbesondere von claudicatio intermittens. Außerdem eignen sich NHE-Inhibitoren somit hervorragend zur Behandlung und Prävention der Herzinsuffizienz, des congestive heart failure (CHF) sowie zur Behandlung und insbesondere zur Prävention von altersbedingten Formen von Krebs.
Dabei kommt eine Kombination mit blutdrucksenkenden Medikamenten, wie mit ACE-Hemmern, Angiotensinrezeptorantagonisten, Diuretika, Ca²⁺-Antagonisten etc. oder mit stoffwechselnormalisierenden Medikamenten, wie Cholesterinsenkern, in Betracht. Die Verbindungen der Formel I eignen sich somit zur Prävention altersbedingter Gewebsveränderungen und zur Gesunderhaltung und Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Weiterhin sind NHE3-Inhibitoren zur Behandlung von durch Bakterien sowie durch Protozoen ausgelösten Erkrankungen (human und veterinär) geeignet. Bei den durch Protozoen ausgelösten Erkrankungen sind insbesondere Malariaerkrankungen des Menschen und Hühnercoccidiose zu nennen.

Außerdem eignen sich die Verbindungen als Mittel zur Bekämpfung von saugenden Parasiten in der Human- und Veterinärmedizin sowie im Pflanzenschutz. Dabei ist die Verwendung als Mittel gegen blutsaugende Parasiten in der Human- und Veterinärmedizin bevorzugt.

Die genannten Verbindungen finden daher vorteilhaft Verwendung allein oder in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie-oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

Beansprucht wird weiterhin ein Heilmittel für die humane, veterinäre oder phytoprotektive Anwendung, enthaltend eine wirksame Menge einer Verbindung der Formel I und/oder deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.

Arzneimittel, die eine Verbindung der Formel I oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin und im Pflanzenschutz. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v. Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

### Versuchsbeschreibungen und Beispiele

Liste der verwendeten Abkürzungen:
- ADMET: Adsorption - Distribution - Metabolismus - Ausscheidung -Toxikologie
- Lsm.: Lösungsmittel
- MeOH: Methanol
- MPRC: Cartridge L-026-30; SI60 40-63µm; Super Vario Flash; max.press. 3 bar Götec-Labortechnik GmbH
- THF: Tetrahydrofuran

### Beispiel 1: 6,8-Dichlor-2-methyl-4-[4N-(2-ethoxy-3,4-dioxocyclobuten-1-yl)amino]phenyl-1,2,3,4-tetrahydroisochinolin

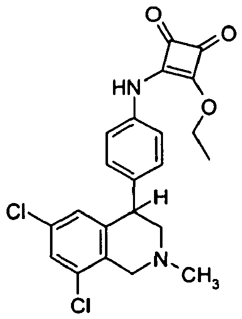

Zu einer Lösung aus 0,58 g 4-(4-Aminophenyl)-6,8-dichlor-2-methyl-1,2,3,4-tetrahydroisochinolin in 40 ml wasserfreien Ethanol gab man 320 mg 1,2-Diethoxy-3,4-dioxo-1-cyclobuten (Quadratsäure-diethylester) und filtrierte den Niederschlag nach Rühren über 48 Stunden bei Raumtemperatur ab. Farbloser kristalliner Feststoff, Zersetzungspunkt: 240°C.

### Beispiel 2: 6,8-Dichlor-2-methyl-4-[3N-(2-ethoxy-3,4-dioxocyclobuten-1-yl)aminoJphenyl-1,2,3,4-tetrahydroisochinolin

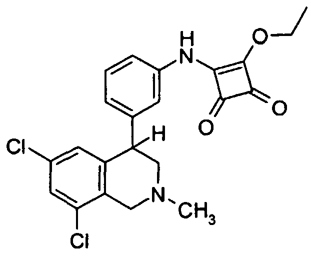

Zu einer Lösung aus 0,125 g 4-(3-Aminophenyl)-6,8-dichlor-2-methyl-1,2,3,4-tetrahydroisochinolin in 40 ml wasserfreien Ethanol gab man 69,2 mg 1,2-Diethoxy-3,4-dioxo-1-cyclobuten (Quadratsäure-diethylester), rührte über 48 Stunden bei Raumtemperatur und destillierte das Lösungsmittel am Rotavapor unter vermindertem Druck ab. Der ölige Rückstand wurde durch MPRC -Chromatographie mit einem Lösungsmittelgemisch aus gleichen Volumenteilen Essigester und Toluol getrennt. Gelber, kristalliner Feststoff, Schmp. 188-194°C.

### Beispiel 3: 6,8-Dichlor-2-methyl-4-[2N-(2-ethoxy-3,4-dioxocyclobuten-1-yl)amino]phenyl-1,2,3,4-tetrahydroisochinolin

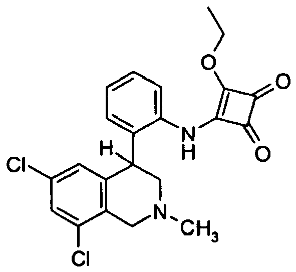

Zu einer Lösung aus 150 mg 4-(2-Aminophenyl)-6,8-dichlor-2-methyl-1,2,3,4-tetrahydroisochinolin in 10 ml wasserfreien Ethanol gab man 0,07 ml 1,2-Diethoxy-3,4-dioxo-1-cyclobuten (Quadratsäure-diethylester), rührte über 70-80 Stunden bei Raumtemperatur und destillierte das Lösungsmittel am Rotavapor unter vermindertem Druck ab. Der Rückstand wurde in wenig Ethylacetat gelöst, ca. 4 Stunden bei Raumtemperatur und eine Nacht im Kühlschrank bei 0-5°C stehen gelassen. Der kristalline Niederschlag wurde abfiltriert. Farbloser, kristalliner Feststoff, Schmp. 152-155°C.

### Beispiel 4: 6,8-Dichlor-2-methyl-4(S)-[4N-(2-ethoxy-3,4-dioxocyclobuten-1-yl)amino]phenyl-1,2,3,4-tetrahydroisochinolin

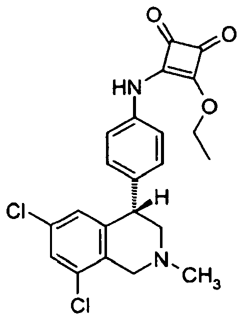

Zu einer Lösung aus 0,51 g 4(S)-(4-Aminophenyl)-6,8-dichlor-2-methyl-1,2,3,4-tetrahydroisochinolin in 40 ml wasserfreien Ethanol gab man 0,28 g 1,2-Diethoxy-3,4-dioxo-1-cyclobuten (Quadratsäure-diethylester) und rührte über 6 Stunden bei Raumtemperatur und destillierte das Lösungsmittel unter vermindertem Druck am Rotavapor ab. Der Rückstand kristallisierte unter Diisopropylether. Farbloser kristalliner Feststoff, Schmp. 208-212°C.

### Beispiel 5: 6,8-Dichlor-2-methyl-4(S)-[3N-(2-ethoxy-3,4-dioxocyclobuten-1-yl)amino]phenyl-1,2,3,4-tetrahydroisochinolin

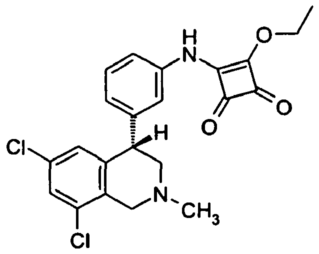

Zu einer Lösung aus 0,366 g 4(S)-(3-Aminophenyl)-6,8-dichlor-2-methyl-1,2,3,4-tetrahydroisochinolin in 25 ml wasserfreien Ethanol gab man 0,2 g 1,2-Diethoxy-3,4-dioxo-1-cyclobuten (Quadratsäure-diethylester) und rührte über 6 Stunden bei Raumtemperatur und destillierte das Lösungsmittel unter vermindertem Druck am Rotavapor ab. Der Rückstand kristallisierte unter Düsopropylether. Farbloser kristalliner Feststoff, Schmp. 198-202°C.

### Beispiel 6: 6,8-Dichlor-2-methyl-4(R)-[2N-(2-ethoxy-3,4-dioxocyclobuten-1-yl)amino]phenyl-1,2,3,4-tetrahydroisochinolin

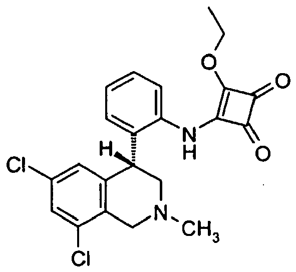

Zu einer Lösung aus 0,64 g 4(R)-(2-Aminophenyl)-6,8-dichlor-2-methyl-1,2,3,4-tetrahydroisochinolin in 40 ml wasserfreien Ethanol gab man 0,309 ml 1,2-Diethoxy-3,4-dioxo-1-cyclobuten (Quadratsäure-diethylester) und rührte über 50-60 Stunden bei Raumtemperatur und destillierte das Lösungsmittel unter vermindertem Druck am Rotavapor ab. Der Rückstand kristallisierte unter Diisopropylether. Farbloser kristalliner Feststoff, Schmp. 205-210°C.

### Beispiel 7: 6,8-Dichlor-2-methyl-4-[4N-(2-hydroxy-3,4-dioxocyclobuten-1-yl)amino]phenyl-1,2,3,4-tetrahydroisochinolin Hydrochlorid

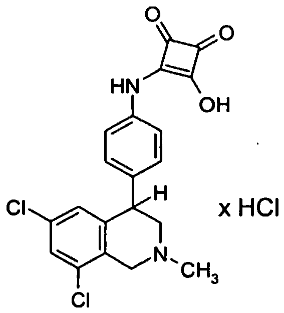

Zu einer Lösung von 100 mg 6,8-Dichlor-2-methyl-4-[4N-(2-ethoxy-3,4-dioxocyclobuten-1-yl)amino]phenyl-1,2,3,4-tetrahydroisochinolin (Beispiel 1) in 15 ml THF wurde eine Lösung von 97,3 mg Lithiumhydroxid Hydrat (LiOHxH₂O) in 15 ml H₂O gegeben und die Mischung über 15 Stunden am Rückflußkühler gekocht. Man verdampfte das THF am Rotavapor und behandelte die wäßrige Lösung mit 2N HCl. Die entstandene Suspension wurde ca 1 Stunde bei Raumtemperatur gerührt und der Niederschlag abfiltriert. Farbloses kristallines Produkt, Schmp. > 310°C.

### Beispiel 8: 6,8-Dichlor-2-methyl-4-[3N-(2-hydroxy-3,4-dioxocyclobuten-1-yl)amino]phenyl-1,2,3,4-tetrahydroisochinolin Hydrochlorid

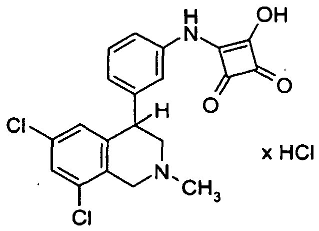

erhielt man analog der in Beispiel 7 beschriebenen Vorschrift aus 6,8-Dichlor-2-methyl-4-[3N-(2-ethoxy-3,4-dioxocyclobuten-1-yl)amino]phenyl-1,2,3,4-tetrahydroisochinolin. Feststoff, der ab 110°C sublimiert.

### Beispiel 9: 6,8-Dichlor-2-methyl-4-[2N-(2-hydroxy-3,4-dioxocyclobuten-1-yl)amino]phenyl-1,2,3,4-tetrahydroisochinolin Hydrochlorid

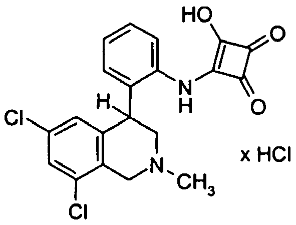

erhielt man analog der in Beispiel 7 beschriebenen Vorschrift aus 6,8-Dichlor-2-methyl-4-[2N-(2-ethoxy-3,4-dioxocyclobuten-1-yl)amino]phenyl-1,2,3,4-tetrahydroisochinolin. Kristalliner Feststoff, Schmp. >310 °C.

### Beispiel 10: 6,8-Dichlor-2-methyl-4-[4N-(2-amino-3,4-dioxocyclobuten-1-yl)amino]phenyl-1,2,3,4-tetrahydroisochinolin

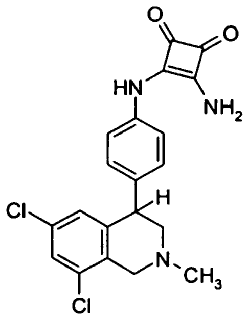

100 mg einer Suspension von 6,8-Dichlor-2-methyl-4-[4N-(2-ethoxy-3,4-dioxocyclobuten-1-yl)amino]phenyl-1,2,3,4-tetrahydroisochinolin (Beispiel 1) in 3 ml Methanol wurden mit 3 ml einer methanolischen Ammoniaklösung 3 Stunden bei Raumtemperatur gerührt, die Kristalle abfiltriert und mit wenig Methanol gewaschen. Farblose kristalline Substanz, Schmp. > 310°C.

### Beispiel 11: 6,8-Dichlor-2-methyl-4-[3N-(2-amino-3,4-dioxocyclobuten-1-yl)amino]phenyl-1,2,3,4-tetrahydroisochinolin

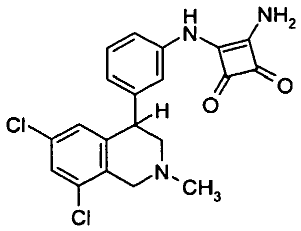

wurde analog der in Beispiel 10 beschriebenen Vorschrift durch Umsetzung von 6,8-Dichlor-2-methyl-4-[3N-(2-ethoxy-3,4-dioxocyclobuten-1-yl)amino]phenyl-1,2,3,4-tetrahydroisochinolin mit methanolischer Ammoniaklösung erhalten. Farblose kristalline Substanz, Schmp. > 310°C.

### Beispiel 12: 6,8-Dichlor-2-methyl-4-[2N-(2-amino-3,4-dioxocyclobuten-1-yl)amino]phenyl-1,2,3,4-tetrahydroisochinolin

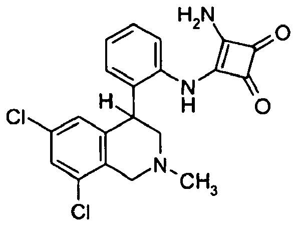

wurde analog der in Beispiel 10 beschriebenen Vorschrift durch Umsetzung von 6,8-Dichlor-2-methyl-4-[2N-(2-ethoxy-3,4-dioxocyclobuten-1-yl)amino]phenyl-1,2,3,4-tetrahydroisochinolin mit methanolischer Ammoniaklösung erhalten. Farblose kristalline Substanz, Zersetzungspunkt: ab 170°C.

### Beispiel 13: 6,8-Dichlor-2-methyl-4(S)-[4N-(2-amino-3,4-dioxocyclobuten-1-yl)amino]phenyl-1,2,3,4-tetrahydroisochinolin

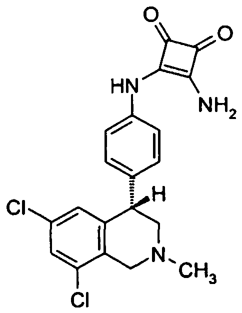

wurde analog der in Beispiel 10 beschriebenen Vorschrift durch Umsetzung von 6,8-Dichlor-2-methyl-4(S)-[4N-(2-ethoxy-3,4-dioxocyclobuten-1-yl)amino]phenyl-1,2,3,4-tetrahydroisochinolin mit methanolischer Ammoniaklösung erhalten. Farblose kristalline Substanz, Schmp.: >310°C.

### Beispiel 14: 6,8-Dichlor-2-methyl-4(S)-[3N-(2-amino-3,4-dioxocyclobuten-1-yl)amino]phenyl-1,2,3,4-tetrahydroisochinolin

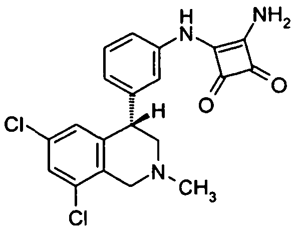

wurde analog der in Beispiel 10 beschriebenen Vorschrift durch Umsetzung von 6,8-Dichlor-2-methyl-4(S)-[3N-(2-ethoxy-3,4-dioxocyclobuten-1-yl)amino]phenyl-1,2,3,4-tetrahydroisochinolin mit methanolischer Ammoniaklösung erhalten. Farblose kristalline Substanz, Zersetzungspunkt: ab 190°C.

### Beispiel 15: 6,8-Dichlor-2-methyl-4(R)-[2N-(2-amino-3,4-dioxocyclobuten-1-yl)amino]phenyl-1,2,3,4-tetrahydroisochinolin

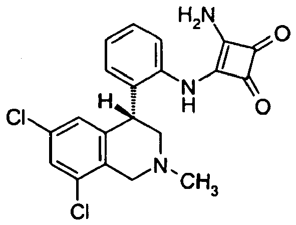

wurde analog der in Beispiel 10 beschriebenen Vorschrift durch Umsetzung von 6,8-Dichlor-2-methyl-4(R)-[2N-(2-ethoxy-3,4-dioxocyclobuten-1-yl)amino]phenyl-1,2,3,4-tetrahydroisochinolin mit methanolischer Ammoniaklösung erhalten. Farblose kristalline Substanz, Schmp. 215-220°C.

### Beispiel 16: Allgemeine Vorschrift zur Herstellung von 4-[N-(2-Amino-3,4-dioxocyclobuten-1-yl)amino]phenyl-1,2,3,4-tetrahydroisochinolinen (XIX)

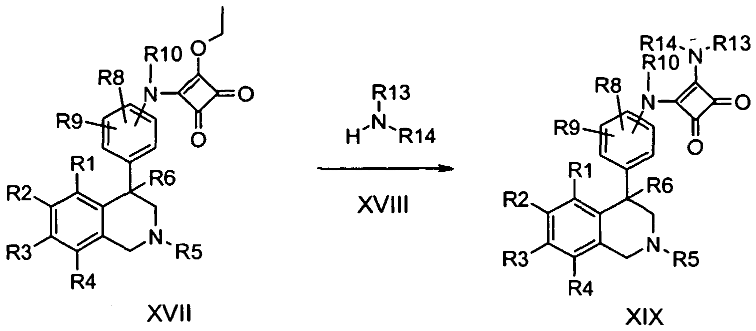

Zu einer Lösung oder Suspension von 1 mmol eines Quadratsäureesters der Formel XVII in 50-100 ml wasserfreiem Methanol gab man 1 - 2 mmol Amin der Formel XVIII und rührte das Reaktionsgemisch über 3-24 Stunden bei Raumtemperatur oder leichtem Erwärmen, wobei der Reaktionsfortschritt dünnschicht-chromatografisch oder mit LC-MS-Analytik verfolgt wurde. Das Endprodukt XIX wurde durch Filtration abgetrennt und mit wenig Methanol gewaschen, wenn die Verbindung als Niederschlag aus der Lösung ausfiel (Variante a).
Wenn das Endprodult Niederschlag nicht als Niederschlag isolierbar war, wurde das Lösungsmittel am Rotavapor unter vermindertem Druck abdestilliert und der zumeist ölige Rückstand unter einem Lösungsmittel, wie z.B. unter Diisopropylether, zur Kristallisation gebracht (Variante b).
In anderen Fällen erfolgte eine Aufreinigung des Rückstands durch präparative MPRC -Chromatographie erfolgen (Variante c), wobei ein Gemisch A) aus 10 Volumenteilen Methylenchlorid oder B) 1 Teil Methanol oder ein Gemisch aus 10 Volumenteilen Ethylacetat, 5 Volumenteilen n-Heptan, 5 Volumenteilen Methylenchlorid, 5 Volumenteilen Methanol und 1 Volumenteil 28%iges Ammoniak verwendet wurde.

Entsprechend dieser Vorschrift wurden die in der nachfolgenden Tabelle angegebenen erfindungsgemäßen Beispiele hergestellt.

| Beispiel | Bezeichnung | Formel XIX | Reaktionsbedingungen | Schmp. °C |
|---|---|---|---|---|
| 17 | 6,8-Dichlor-2-methyl-4-[4N-(2-N-cyclohexyl-N-methylamino-3,4-dioxo-cyclobuten-1-yl)amino]phenyl-1,2,3,4-tetrahydroisochinolin | 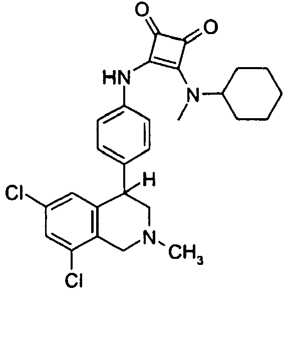 | Variante a | 260-265 |
| 18 | 6,8-Dichlor-2-methyl-4-[4N-(2-dimethylamino-3,4-dioxocyclobuten-1-yl)amino]phenyl-1,2,3,4-tetrahydroisochinolin | 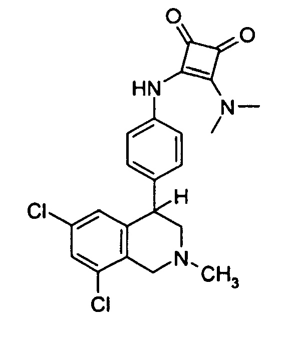 | Variante b, Kristallisation aus Ethylacetat | 208-214 |
| 19 | 6,8-Dichlor-2-methyl-4-[4N-(2-diethylamino-3,4-dioxocyclobuten-1-yl)amino]phenyl-1,2,3,4-tetrahydroisochinolin | 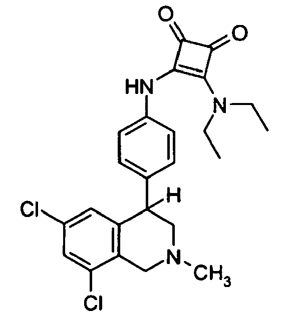 | Variante b, Kristallisation aus einer Mischung aus Ethylacetat und Diiso-propylether | 275-280 |
| 20 | 6,8-Dichlor-2-methyl-4(S)-[4N-(2-dimethylamino-3,4-dioxocyclobuten-1-yl)amino]phenyl-1,2,3,4-tetrahydroisochinolin | 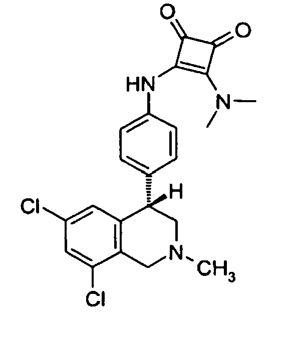 | Variante b, Kristallisation aus Ethylacetat | 240-247 |
| 21 | 6,8-Dichlor-2-methyl-4(R)-[2N-(2-dimethylamino-3,4-dioxocyclobuten-1-yl)amino]phenyl-1,2,3,4-tetrahydroisochinolin | 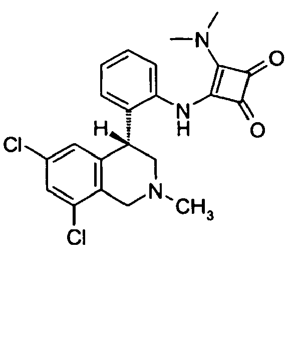 | Variante c, MPRC in B), anschließend Kristallisation aus Ethylacetat | 236-240 |
| 22 | 6,8-Dichlor-2-methyl-4(S)-[3N-(2-dimethylamino-3,4-dioxocyclobuten-1-yl)amino]phenyl-1,2,3,4-tetrahydroisochinolin | 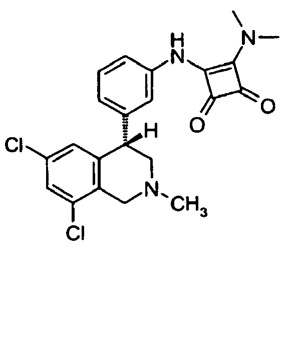 | Variante c, MPRC in B), anschließend Kristallisation aus Ethylacetat | Zersetz ungspunkt 140 °C |
| 23 | 6,8-Dichlor-2-methyl-4(S)-[4N-(2N-(1-butyl)-N-methylamino-3,4-dioxocyclobuten-1-yl)amino]phenyl-1,2,3,4-tetrahydroisochinolin | 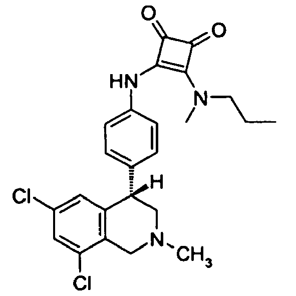 | Variante c, MPRC in B), anschließend Kristallisation aus Diisopropylether | 145-150 |
| 24 | 6,8-Dichlor-2-methyl-4-[4N-(2-dipropylamino-3,4-dioxocyclobuten-1-yl)amino]phenyl-1,2,3,4-tetrahydroisochinolin | 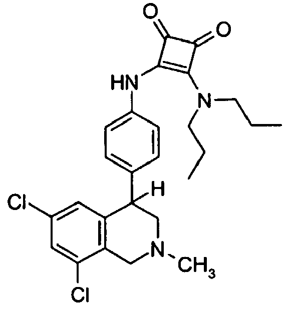 | Variante c, MPRC in B) | Zersetz ungspunkt ab 130°C |
| 25 | 6,8-Dichlor-2-methyl-4-[4N-(2-(N-isobutyl-N-methylamino)-3,4-dioxocyclobuten-1-yl)amino]phenyl-1,2,3,4-tetrahydroisochinolin | 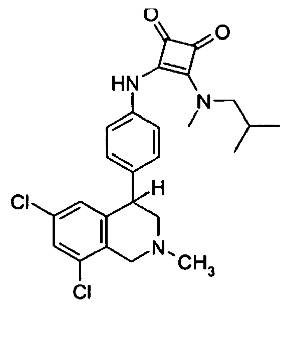 | Variante c, MPRC in B) | > 310 |
| 26 | 6,8-Dichlor-2-methyl-4-[4N-(2-(N-methoxyethyl)-N-methylamino)-3,4-dioxocyclobuten-1-yl)amino]phenyl-1,2,3,4-tetrahydroisochinolin | 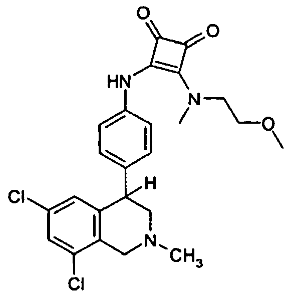 | Variante c, MPRC in B), anschließend Kristallisation aus Ethylacetat | 206-210 |
| 27 | 6,8-Dichlor-2-methyl-4(S)-[4N-(2-diethylamino-3,4-dioxocyclobuten-1-yl)amino]phenyl-1,2,3,4-tetrahydroisochinolin | 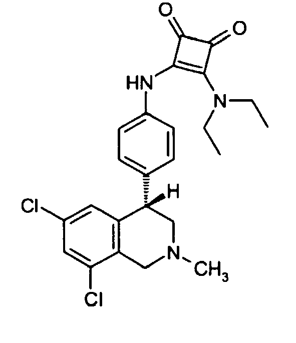 | Variante c, MPRC in B), anschließend Kristallisation aus Diisopropylether/ Ethylacetat | 220-225 |
| 28 | 6,8-Dichlor-2-methyl-4(S)-[4N-(2-(N-Isopropyl-N-methylamino)-3,4-dioxocyclobuten-1-yl)amino]phenyl-1,2,3,4-tetrahydroisochinolin | 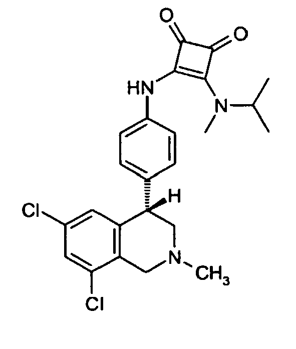 | Variante c, MPRC in B), anschließend Kristallisation aus Diisopropylether/ Ethylacetat | 165-175 (Zersetz ung) |
| 29 | 6,8-Dichlor-2-methyl-4-[4N-(2-(N-cyclopropyl-methyl-N-propylamino)-3,4-dioxocyclobuten-1-yl)amino]-phenyl-1,2,3,4-tetrahydroisochinolin | 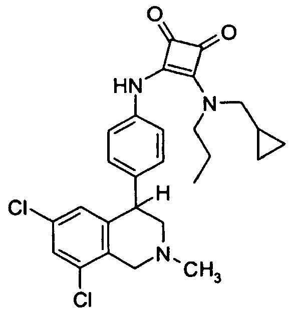 | Variante c, MPRC in A), anschließend Kristallisation aus Wasser | 134-144 |
| 30 | 6,8-Dichlor-2-methyl-4(S)-[3N-(2-diethylamino-3,4-dioxocyclobuten-1-yl)amino]phenyl-1,2,3,4-tetrahydroisochinolin | 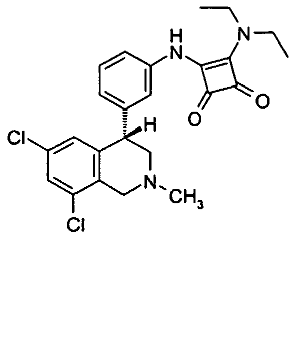 | Variante c, MPRC in A), anschließend Kristallisation aus Wasser | 125-135 |
| 31 | 6,8-Dichlor-2-methyl-4(S)-[3N-(2-N-methyl-N-isopropyl)amino-3,4-dioxocyclobuten-1-yl)amino]phenyl-1,2,3,4-tetrahydroisochinolin | 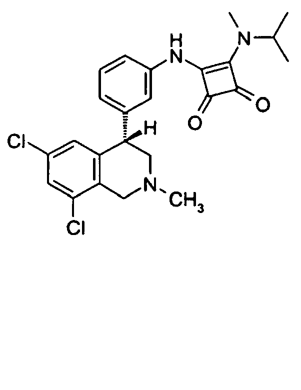 | Variante c, MPRC in A), anschließend Kristallisation aus Wasser | 135-150 |
| 32 | 6,8-Dichlor-2-methyl-4(S)-[4N-(2N-ethyl-N-isopropylamino)-3,4-dioxocyclobuten-1-yl)amino]phenyl-1,2,3,4-tetrahydroisochinolin | 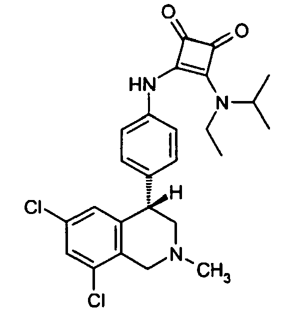 | Variante c, MPRC in B), anschließend Kristallisation aus Diiso-propylether/ Ethylacetat, anschließend aus Wasser | 160-190 |
| 33 | 6,8-Dichlor-2-methyl-4(S)-[3N-(2-N-methyl-N-propylamino)-3,4-dioxocyclobuten-1-yl)amino]phenyl-1,2,3,4-tetrahydroisochinolin | 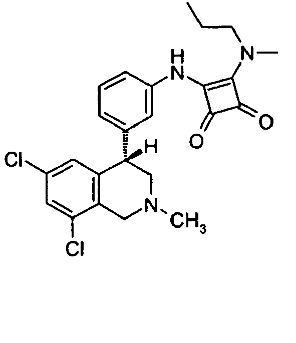 | Variante c, MPRC in A), anschließend Kristallisation aus Wasser | 135-140 |
| 34 | 6,8-Dichlor-2-methyl-4(S)-[3N-(2-N-pyrrolidino-3,4-dioxocyclobuten-1-yl)amino]phenyl-1,2,3,4-tetrahydroisochinolin | 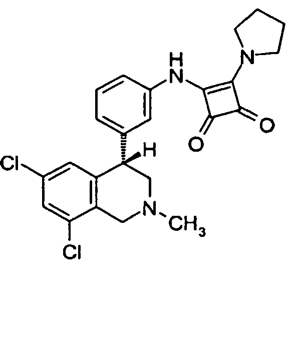 | Variante c, MPRC in A), anschließend Kristallisation aus Wasser | 138-143 |
| 35 | 6,8-Dichlor-2-methyl-4(S)-[4N-(2-N-cyclopropylamino)-3,4-dioxo-cyclobuten-1-yl)amino]phenyl-1,2,3,4-tetrahydroisochinolin | 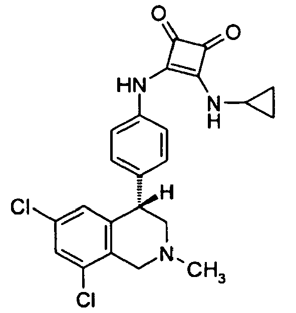 | Variante a | 280-285 |
| 36 | 6,8-Dichlor-2-methyl-4(S)-[4N-(2-N-pyrrolidino-3,4-dioxocyclobuten-1-yl)amino]phenyl-1,2,3,4-tetrahydroisochinolin | 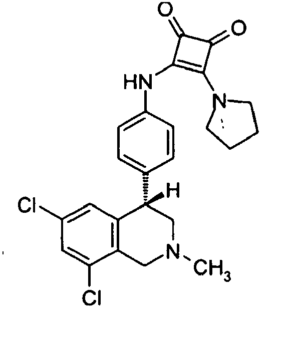 | Variante a | 264-270 |
| 37 | 6,8-Dichlor-2-methyl-4(R)-[2N-(2-N-methyl-N-propylamino)-3,4-dioxocyclobuten-1-yl)amino]phenyl-1,2,3,4-tetrahydroisochinolin | 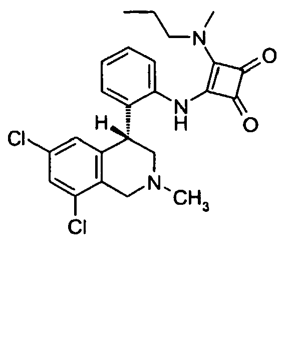 | Variante a | 236-240 |
| 38 | 6,8-Dichlor-2-methyl-4-[4N-(2-N-benzyl-N-methylamino)-3,4-dioxo-cyclobuten-1-yl)amino]phenyl-1,2,3,4-tetrahydroisochinolin | 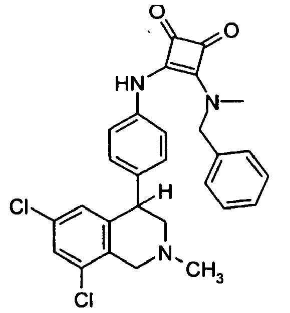 | Rückstand mit Wasser versetzt, mit Ethylacetat extrahiert, Variante c, MPRC in A), anschließend Kristallisation aus Wasser | 158-162 |
| 39 | 6,8-Dichlor-2-methyl-4(R)-[2N-(2-diethylamino-3,4-dioxocyclobuten-1-yl)amino]phenyl-1,2,3,4-tetrahydroisochinolin | 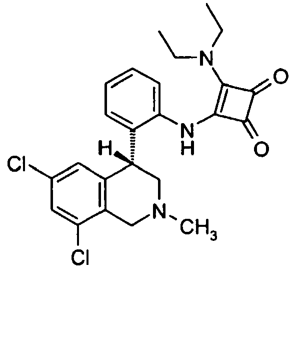 | Variante b, Kristallisation aus Ethylacetat | 246-250 |
| 40 | 6,8-Dichlor-2-methyl-4(R)-[2N-(2-N,N-dipropylamino)-3,4-dioxocyclobuten-1-yl)amino]phenyl-1,2,3,4-tetrahydroisochinolin | 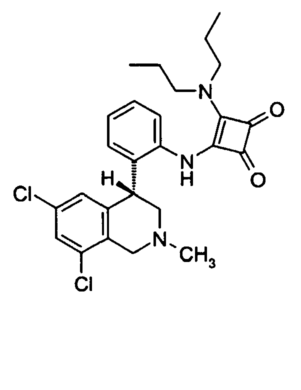 | Variante c, MPRC in A), anschließend Kristallisation aus Wasser | 190-194 |
| 41 | 6,8-Dichlor-2-methyl-4(R)-[2N-(2-N-pyrrolidino-3,4-dioxocyclobuten-1-yl)amino]phenyl-1,2,3,4-tetrahydroisochinolin | 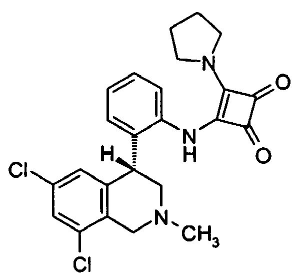 | Variante c, MPRC in A), anschließend Kristallisation aus Wasser | 220-224 |
| 42 | 6,8-Dichlor-2-methyl-4(R)-[2N-(2-N-methyl-N-isopropyl)amino-3,4-dioxocyclobuten-1-yl)amino]phenyl-1,2,3,4-tetrahydroisochinolin | 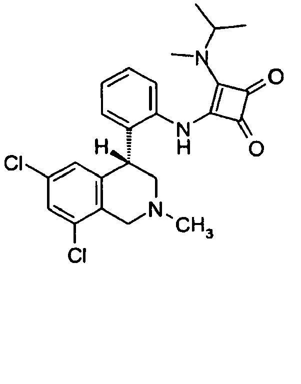 | Variante c, MPRC in A), anschließend Kristallisation aus Wasser | 210-214 |
| 43 | 6,8-Dichlor-2-methyl-4-[4N-(2-N-phenylamino-3,4-dioxocyclobuten-1-yl)amino]phenyl-1,2,3,4-tetrahydroisochinolin | 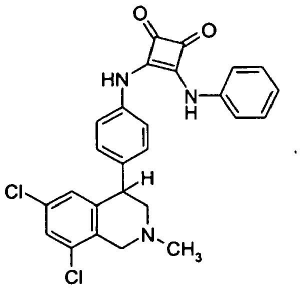 | Variante b, Kristallisation aus Ethylacetat | 288-294 |
| 44 | 6,8-Dichlor-2-methyl-4-[2N-(2-N-methylamino-3,4-dioxocyclobuten-1-yl)amino]phenyl-1,2,3,4-tetrahydroisochinolin | 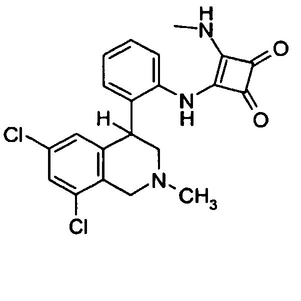 | Variante c, MPRC in A), anschließend Kristallisation aus wenig Ethylacetat | 224-230 |
| 45 | 6,8-Dichlor-2-methyl-4-[2N-(2-N-(1-hexylamino)-3,4-dioxocyclobuten-1-yl)amino]phenyl-1,2,3,4-tetrahydroisochinolin | 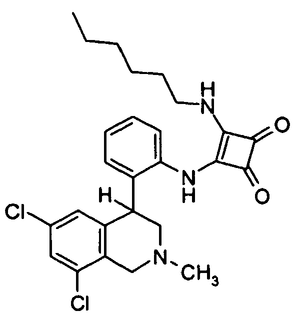 | Variante c, MPRC in A), anschließend Kristallisation aus wenig Ethylacetat | 157-160 |
| 46 | 6,8-Dichlor-2-methyl-4-[2N-(2-N-isopropylamino-3,4-dioxocyclobuten-1-yl)amino]phenyl-1,2,3,4-tetrahydroisochinolin | 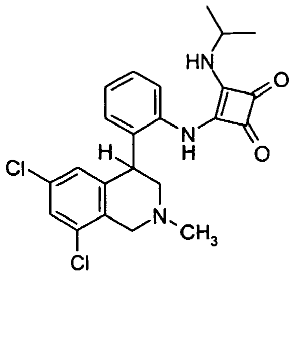 | Variante c, MPRC in A), anschließend Kristallisation aus wenig Ethylacetat | 238-241 |
| 47 | 6,8-Dichlor-2-methyl-4-[2N-(2-N-cyclopentylamino-3,4-dioxocyclobuten-1-yl)amino]phenyl-1,2,3,4-tetrahydroisochinolin | 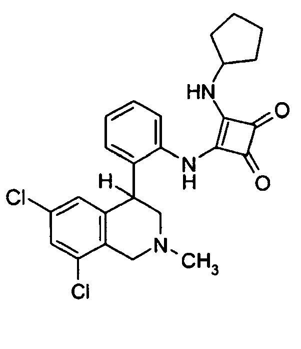 | Variante a | 278-281 |
| 48 | 6,8-Dichlor-2-methyl-4-[2N-(2N-(2-furylmethyl)amino-3,4-dioxocyclobuten-1-yl)amino]phenyl-1,2,3,4-tetrahydroisochinolin | 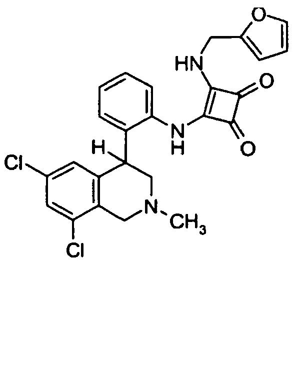 | Variante c, MPRC in A), anschließend Kristallisation aus wenig Ethylacetat | 279-283 |
| 49 | 6,8-Dichlor-2-methyl-4-[2N-(2-N-ethylamino-3,4-dioxocyclobuten-1-yl)amino]phenyl-1,2,3,4-tetrahydroisochinolin | 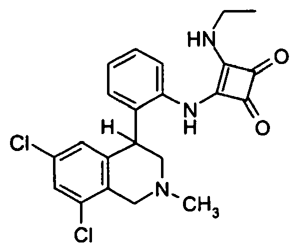 | Variante c, MPRC in A), anschließend Kristallisation aus wenig Ethylacetat | 205-208 |
| 50 | 6,8-Dichlor-2-methyl-4-[2N-(2-N-dimethylaminoethy lamino-3,4-dioxocyclobuten-1-yl)amino]phenyl-1,2,3,4-tetrahydroisochinolin | 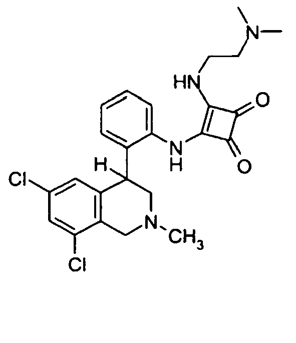 | Variante c, MPRC in A), anschließend Kristallisation aus wenig Ethylacetat | 208-211 |
| 51 | 6,8-Dichlor-2-methyl-4-[4N-(2-N-ethylamino-3,4-dioxocyclobuten-1-yl)amino]phenyl-1,2,3,4-tetrahydroisochinolin | 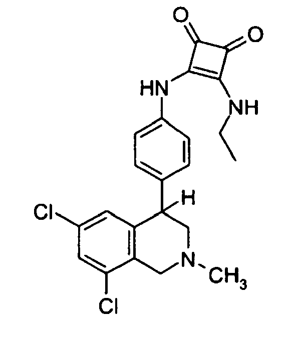 | Variante a | 292-297 |
| 52 | 6,8-Dichlor-2-methyl-4-[2N-(2N-(3-picolylmethyl)amin o-3,4-dioxocyclobuten-1-yl)amino]phenyl-1,2,3,4-tetrahydroisochinolin | 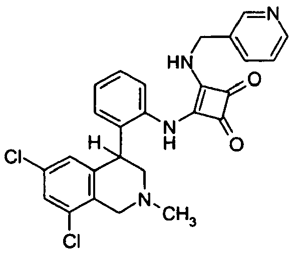 | Variante c, MPRC in A), anschließend Kristallisation aus wenig Ethylacetat | 244-246 |
| 53 | 6,8-Dichlor-2-methyl-4-[4N-(2N-methylamino-3,4-dioxocyclobuten-1-yl)amino]phenyl-1,2,3,4-tetrahydroisochinolin | 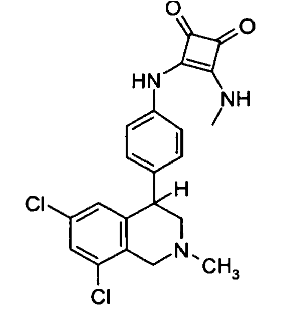 | Variante a | 328-334 |
| 54 | 6,8-Dichlor-2-methyl-4-[4N-(2N-cyclopropylamino-3,4-dioxocyclobuten-1-yl)amino]phenyl-1,2,3,4-tetrahydroisochinolin | 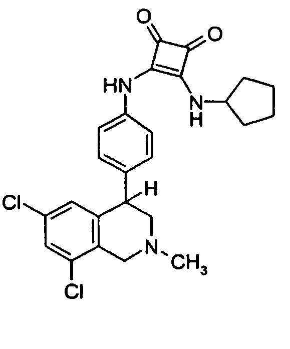 | Variante a | 300-304 |
| 55 | 6,8-Dichlor-2-methyl-4-[4N-(2N-isopropylamino-3,4-dioxocyclobuten-1-yl)amino]phenyl-1,2,3,4-tetrahydroisochinolin | 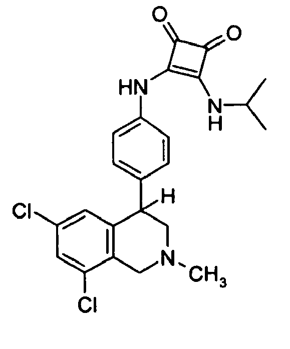 | Variante a | 268-272 |
| 56 | 6,8-Dichlor-2-methyl-4-[4N-(2N-(2-furylmethyl)-amino-3,4-dioxocyclobuten-1-yl)amino]phenyl-1,2,3,4-tetrahydroisochinolin | 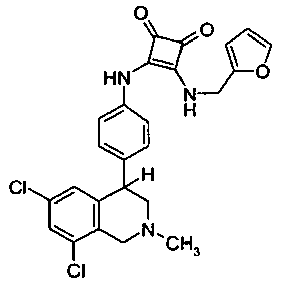 | Variante a | 264-268 |
| 57 | 6,8-Dichlor-2-methyl-4-[4N-(2N-dimethylaminoethy lamino)-3,4-dioxocyclobuten-1-yl)amino]phenyl-1,2,3,4-tetrahydroisochinolin | 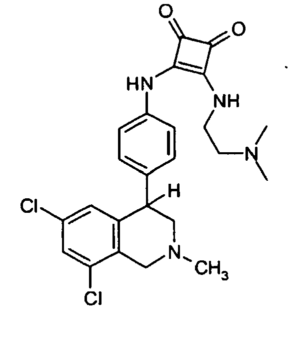 | Variante a | 262-266 |
| 58 | 6,8-Dichlor-2-methyl-4-[4N-(1-hexylamino)-3,4-dioxocyclobuten-1-yl)amino]phenyl-1,2,3,4-tetrahydroisochinolin | 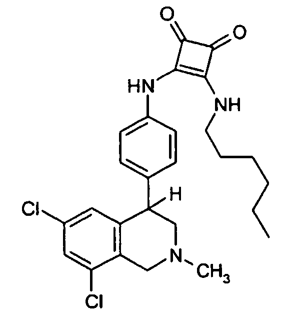 | Variante a | 234-238 |
| 59 | 6,8-Dichlor-2-methyl-4-[4N-(3-picolylamino)-3,4-dioxo-cyclobuten-1-yl)amino]phenyl-1,2,3,4-tetrahydroisochinolin | 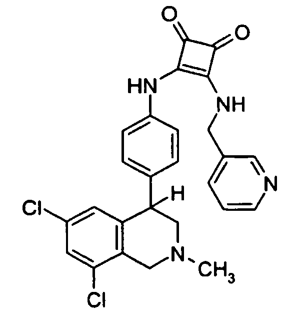 | Variante a | 288-290 |
| 60 | 6,8-Dichlor-2-methyl-4-[4N-2-methylamino-3,4-dioxo-cyclobuten-1-yl)amino]phenyl-1,2,3,4-tetrahydroisochinolin | 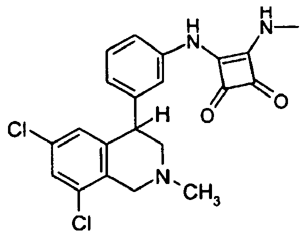 | Variante a | 295-298 |
| 61 | 6,8-Dichlor-2-methyl-4-[4N-(2-ethylamino)-3,4-dioxo-cyclobuten-1-yl)amino]phenyl-1,2,3,4-tetrahydroisochinolin | 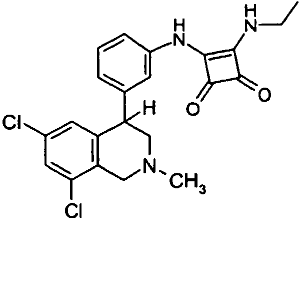 | Variante a | Ab 200 °C Zersetzung |
| 62 | 6,8-Dichlor-2-methyl-4-[3N-(2-(2-furylmethyl-amino)-3,4-dioxo-cyclobuten-1-yl)amino]phenyl-1,2,3,4-tetrahydroisochinolin | 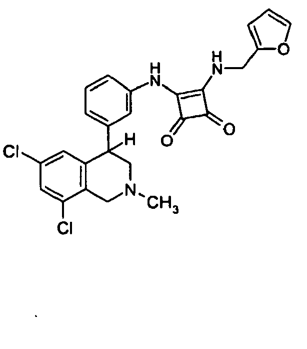 | Variante a | Ab 200 °C Zersetzung |
| 63 | 6,8-Dichlor-2-methyl-4-[3N-(2-cyclopentylamino-3,4-dioxo-cyclobuten-1-yl)amino]phenyl-1,2,3,4-tetrahydroisochinolin | 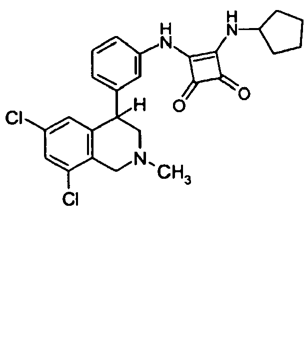 | Variante a | 248-252 |
| 64 | 6,8-Dichlor-2-methyl-4-[3N-(2-isopropylamino)-3,4-dioxo-cyclobuten-1-yl)amino]phenyl-1,2,3,4-tetrahydroisochinolin | 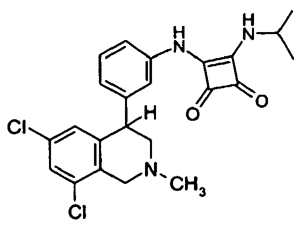 | Variante a | 240-244 |
| 65 | 6,8-Dichlor-2-methyl-4-[3N-(2N-dimethylaminoethy lamino)-3,4-dioxocyclobuten-1-yl)amino]phenyl-1,2,3,4-tetrahydroisochinolin | 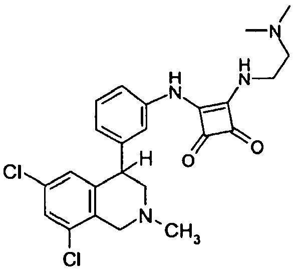 | Variante a | 198-200 |

### Beispiel 66 : 6,8-Dichlor-2-methyl-4-[4-acetylamino-2-methyl-phenyl]-1,2,3,4-tetrahydroisochinolin

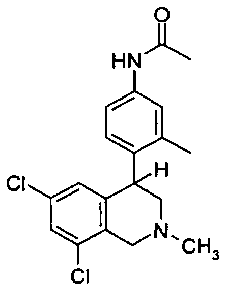

erhielt man durch Zutropfen von 0,4 ml konzentrierter Schwefelsäure (96%) zu einer Lösung von 125 mg 1-(4-Acetylamino-2-methylphenyl)-2-[N-(2,4-dichlorbenzyl)-N-methyl-amino]-ethanol in 3 ml wasserfreiem Dichlormethan. Das Zweiphasengemisch wurde 5 Stunden bei Raumtemperatur gerührt, sodann auf Eis gegossen, mit 2N wäßriger NaOH stark alkalisch gestellt und mehrfach mit Dichlormethan extrahiert. Nach dem Waschen der organischen Phase mit Wasser und Trocknen über Natriumsulfat destillierte man das Lösungsmittel ab, chromatografierte den Rückstand auf der Säule an Kieselgel mit einem Gemisch aus gleichen Teilen Ethylacetat und Toluol und destillierte erneut das Lösungsmittel am Rotavapor unter vermindertem Druck. Kristalliner Feststoff, Schmp. 182-185°C.

### Beispiel 67: 4-[4-Amino-2-methyl-phenyl]-6,8-dichlor-2-methyl-1,2,3,4-tetrahydroisochinolin Dihydrochlorid

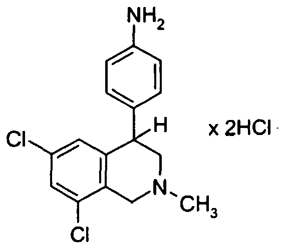

erhielt man durch Kochen einer Suspension von 0,05 g 6,8-Dichlor-2-methyl-4-[4-acetylamino-2-methyl-phenyl]-1,2,3,4-tetrahydroisochinolin (Beispiel 66) in einer Mischung aus 2 ml Wasser und 2 ml konzentrierter Salzsäure über 2 Stunden und Stehenlassen über Nacht bei Raumtemperatur, wobei eine klare Lösung erhalten wurde. Nach dem Abdestillieren der wässrigen Säure erhielt man das Bishydrochlorid als farblose kristalline Verbindung. Schmp (Zersetzung) 250 - 260 °C.
Das 6,8-Dichlor-2-methyl-4-[4-amino-2-methyl-phenyl]-1,2,3,4-tetrahydroisochinolin erhielt man als freie Base durch Behandeln einer wäßrigen Lösung von 6,8-Dichlor-2-methyl-4-[4-amino-2-methyl-phenyl]-1,2,3,4-tetrahydroisochinolin Dihydrochlorid mit NaOH, indem man den Niederschlag filtrierte, mit Wasser wusch und trocknete. Amorpher Feststoff mit Zersetzung ab 70°C.

### Beispiel 68: 6,8-Dichlor-2-methyl-4-[4N-(2-ethoxy-3,4-dioxocyclobuten-1-yl)amino-2-methyl-phenyl]-1,2,3,4-tetrahydroisochinolin

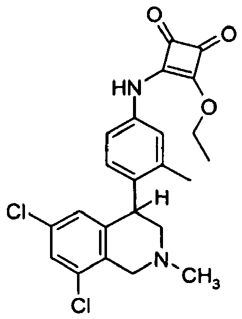

Zu einer Lösung aus 0,2 g 4-[4-Amino-2-methyl-phenyl]-6,8-dichlor-2-methyl-1,2,3,4-tetrahydroisochinolin Dihydrochlorid in 15 ml wasserfreien Ethanol gab man 110 mg 1,2-Diethoxy-3,4-dioxo-1-cyclobuten (Quadratsäure-diethylester) und filtrierte den Niederschlag nach Rühren über 20-25 Stunden bei Raumtemperatur ab. Farbloser kristalliner Feststoff, Schmp.: 235 - 240°C.

### Beispiel 69: 4-[4N-(2-Amino-3,4-dioxocyclobuten-1-yl)amino-2-methyl-phenyl]-6,8-dichlor-2-methyl-1,2,3,4-tetrahydroisochinolin

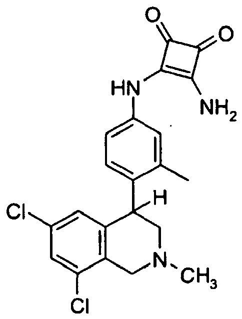

erhielt man durch Zugabe von 4 ml einer gesättigten Lösung von Ammoniak in Methanol zu einer Suspension von 100 mg 6,8-Dichlor-2-methyl-4-[4N-(2-ethoxy-3,4-dioxocyclobuten-1-yl)amino-2-methyl-phenyl]-1,2,3,4-tetrahydroisochinolin (Beispiel 68). Nach 5 Stunden Rühren bei Raumtemperatur und Stehenlassen über Nacht filtrierte man die Kristalle ab und wusch mit Methanol nach. Schmp. 315-318 °C.

### Beispiel 70: 6,8-Dichlor-2-methyl-4-[4N-(2N-methyl-2N-propylamino-3,4-dioxocyclobuten-1-yl)amino-2-methyl-phenyl]-1,2,3,4-tetrahydroisochinolin

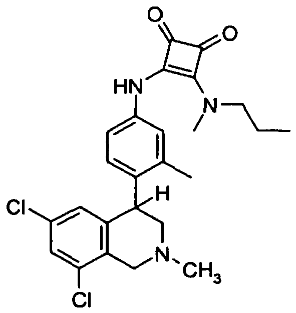

erhielt man analog der in Beispiel 16 angegebenen Vorschrift aus 6,8-Dichlor-2-methyl-4-[4N-(2-ethoxy-3,4-dioxocyclobuten-1-yl)amino-2-methyl-phenyl]-1,2,3,4-tetrahydroisochinolin und N-1-Propyl-N-methylamin. Kristalliner Feststoff, Schmp. 158 -163°C.

### Beispiel 71: 6,8-Dichlor-2-methyl-4-[4N-(2N-methylamino-3,4-dioxocyclobuten-1-yl)amino-2-methyl-phenyl]-1,2,3,4-tetrahydroisochinolin

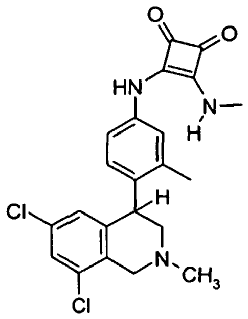

erhielt man analog der in Beispiel 16 angegebenen Vorschrift aus 6,8-Dichlor-2-methyl-4-[4N-(2-ethoxy-3,4-dioxocyclobuten-1-yl)amino-2-methyl-phenyl]-1,2,3,4-tetrahydroisochinolin und Methylamin. Kristalliner Feststoff, Schmp. > 310 °C.

### Beispiel 72: 6,8-Dichlor-2-methyl-4(R)-[3N-(2-methylamino-3,4-dioxocyclobuten-1-yl)amino]phenyl-1,2,3,4-tetrahydroisochinolin

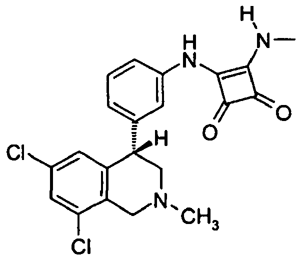

erhielt man analog der in Beispiel 16 angegebenen Vorschrift aus 6,8-Dichlor-2-methyl-4(S)-[3N-(2-ethoxy-3,4-dioxocyclobuten-1-yl)amino]phenyl-1,2,3,4-tetrahydroisochinolin (Beispiel 5) und Methylamin. Kristalliner Feststoff, Schmp. > 310 °C.

### Beispiel 73: 6,8-Dichlor-2-methyl-4(R)-[2N-(2-isopropylamino-3,4-dioxocyclobuten-1-yl)amino]phenyl-1,2,3,4-tetrahydroisochinolin

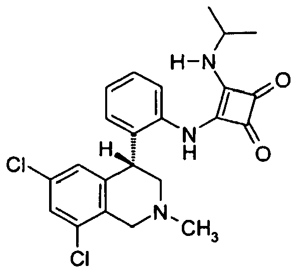

erhielt man analog der in Beispiel 16 angegebenen Vorschrift aus 6,8-Dichlor-2-methyl-4(R)-[2N-(2-ethoxy-3,4-dioxocyclobuten-1-yl)amino]phenyl-1,2,3,4-tetrahydroisochinolin (Beispiel 6) und Isopropylamin. Kristalliner Feststoff, Schmp. 268 - 270 °C.

### Beispiel 74: 6,8-Dichlor-2-methyl-4(R)-[2N-(2-methylamino-3,4-dioxocyclobuten-1-yl)amino]phenyl-1,2,3,4-tetrahydroisochinolin

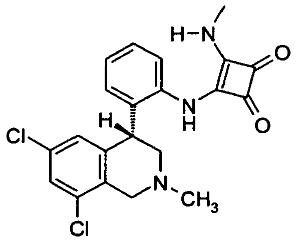

erhielt man analog der in Beispiel 16 angegebenen Vorschrift aus 6,8-Dichlor-2-methyl-4(R)-[2N-(2-ethoxy-3,4-dioxocyclobuten-1-yl)amino]phenyl-1,2,3,4-tetrahydroisochinolin (Beispiel 6) und Methylamin. Kristalliner Feststoff, Schmp. 265°C.

### Pharmakologische Daten:

### Testbeschreibung: Bestimmung der NHE-inhibitorischen Wirkung

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wurde die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl -Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wurde ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCl, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wurde ein pH von 7,0 eingestellt). Für die Bestimmung des 0%-Wertes wurden die Zellen in einem Na⁺-freien Puffer inkubiert (133,8 mM Cholinchlorid, 4,7 mM KCl, 1,25 mM CaCl₂, 1,25 mM MgCl₂, 0,97 mM K₂HPO₄, 0,23 mM KH₂PO₄, 5 mM Hepes, 5 mM Glucose, mit 1 M NaOH wurde ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

| Beispiel | IC50 (µM) |
|---|---|
| 18 | 0.26 |
| 19 | 1,15 |
| 22 | 0,0985 |
| 23 | 0.31 |
| 24 | 2,49 |
| 25 | 2,81 |
| 26 | 1,70 |
| 27 | 0,21 |
| 28 | 0,26 |
| 29 | 7,14 |
| 30 | 0,14 |
| 31 | 0,13 |
| 32 | 0,68 |
| 33 | 0,031 |
| 34 | 0,052 |
| 36 | 0.23 |
| 37 | 0.85 |
| 38 | 8.0 |
| 39 | 1,52 |
| 40 | 0,24 |
| 41 | 0,11 |
| 42 | 0,15 |

## Patentansprüche

1. Verbindungen der Formel I 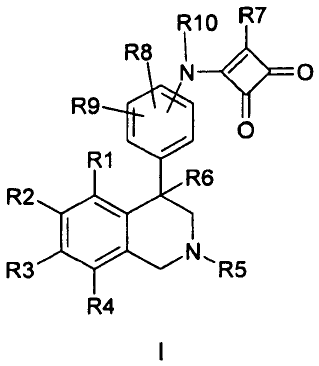 worin bedeuten:
R1, R2, R3 und R4 unabhängig voneinander Wasserstoff, F, Cl, Br, I, CN, NO₂ oder R11-(CₘH₂ₘ)-Aₙ-;
m Null, 1, 2, 3 oder 4;
n Null oder 1;
R11 Wasserstoff, Methyl, CₚF₂ₚ₊₁ oder Phenyl;
p 1, 2 oder 3;
A Sauerstoff, NH, N(CH₃) oder S(O)q;
q Null, 1 oder 2;
R5 Wasserstoff, Alkyl mit 1, 2, 3, 4, 5 oder 6 C-Atomen, das teilweise oder vollständig fluoriert sein kann, oder Cycloalkyl mit 3, 4, 5 oder 6 C-Atomen;
R6 Wasserstoff, OH, F, CF₃, Methyl, Ethyl, Isopropyl oder Cyclopropyl;
R7 Wasserstoff, Alkyl mit 1, 2, 3, 4, 5 oder 6 C-Atomen, Cycloalkyl mit 3, 4, 5 oder 6 C-Atomen, OR12 oder NR13R14;
R12 Wasserstoff, Alkyl mit 1, 2, 3, 4, 5 oder 6 C-Atomen, das teilweise oder vollständig fluoriert sein kann, oder Cycloalkyl mit 3, 4, 5 oder 6 C-Atomen;
R13 und R14 unabhängig voneinander Wasserstoff, Cycloalkyl mit 3, 4, 5 oder 6 C-Atomen, Alkyl mit 1, 2, 3, 4, 5 oder 6 C-Atomen, das teilweise oder vollständig fluoriert sein kann, Phenyl, Phenylalkyl mit 1, 2 oder 3 C-Atomen im Alkylteil, Heteroaryl oder Heteroarylmethyl, wobei Heteroarylreste aromatische Ringverbindungen sind, in denen ein oder mehrere Ringatome Sauerstoffatome, Schwefelatome oder Stickstoffatome sind, bevorzugt 1, 2, 3 oder 4 Stickstoffatome, 1 oder 2 Sauerstoffatome, 1 oder 2 Schwefelatome oder eine Kombinationen aus verschiedenen Heteroatomen und wobei weiterhin die Heteroarylreste über alle Positionen angebunden sein können, wobei die Phenyl- und Heteroarylreste unsubstituiert sind oder substituiert sind mit 1, 2, 3, 4 oder 5 Resten ausgewählt aus der Gruppe bestehend aus Fluor, Chlor, Methyl, CF₃, Methoxy und OH;
wobei die Alkylreste unsubstituiert sind oder substituiert sind mit 1, 2 oder 3 Resten ausgewählt aus der Gruppe Alkoxy mit 1, 2, 3 oder 4 C-Atomen, NR15R16 und Cycloalkyl mit 3, 4, 5 oder 6 C-Atomen;
R15 und R16 unabhängig voneinander Wasserstoff oder Alkyl mit 1, 2, 3 oder 4 C-Atomen;
oder
R13 und R14 zusammen mit dem N-Atom, über das sie miteinander verbunden sind, einen 3, 4, 5, 6, 7, 8 oder 9-gliedrigen Ring, wobei ein C-Atom des Rings durch ein Sauerstoffatom oder eine NCH₃-Gruppe ersetzt sein kann,
R8 und R9 unabhängig voneinander Wasserstoff, F, Cl, OH, CH₃, CH₃O, CF₃, CF₃CH₂O oder CH₃SO₂;
R10 Wasserstoff, Methyl oder Ethyl;
sowie deren pharmazeutisch verträgliche Salze und Trifluoracetate.

2. Verbindungen der Formel I nach Anspruch 1, worin bedeuten
R1, R2, R3 und R4 unabhängig voneinander Wasserstoff, F, Cl, Br, CN oder R11-(CₘH₂ₘ)-Aₙ-;
m Null oder 1;
n Null oder 1;
R11 Wasserstoff, Methyl, CₚF₂ₚ₊₁- oder Phenyl;
p 1 oder 2;
A Sauerstoff oder S(O)q;
q Null, 1 oder 2;
R5 Wasserstoff, Methyl, Ethyl oder Cyclopropyl;
R6 Wasserstoff oder Methyl;
R7 OR12 oder NR13R14;
R12 Wasserstoff oder Alkyl mit 1, 2 oder 3 C-Atomen;
R13 und R14 unabhängig voneinander Wasserstoff, Alkyl mit 1, 2, 3, 4, 5 oder 6 C-Atomen, das teilweise oder vollständig fluoriert sein kann, Cycloalkyl mit 3, 4, 5 oder 6 C-Atomen, Phenyl, Phenylalkyl mit 1, 2 oder 3 C-Atomen im Alkylteil, Heteroaryl oder Heteroarylmethyl; wobei Heteroarylreste aromatische Ringverbindungen sind, in denen ein oder mehrere Ringatome Sauerstoffatome, Schwefelatome oder Stickstoffatome sind, bevorzugt 1, 2, 3 oder 4 Stickstoffatome, 1 oder 2 Sauerstoffatome, 1 oder 2 Schwefelatome oder eine Kombinationen aus verschiedenen Heteroatomen und wobei weiterhin die Heteroarylreste über alle Positionen angebunden sein können, wobei die Phenyl- und Heteroarylreste unsubstituiert sind oder substituiert sind mit 1, 2, 3, 4 oder 5 Resten ausgewählt aus der Gruppe bestehend aus Fluor, Chlor, Methyl, CF₃, Methoxy und OH;
wobei die Alkylreste unsubstituiert sind oder substituiert sind mit 1, 2 oder 3 Resten ausgewählt aus der Gruppe Alkoxy mit 1, 2, 3 oder 4 C-Atomen, NR15R16 und Cycloalkyl mit 3, 4, 5 oder 6 C-Atomen;
R15 und R16 unabhängig voneinander Wasserstoff oder Alkyl mit 1, 2, 3 oder 4 C-Atomen;
oder
R13 und R14 zusammen mit dem N-Atom, über das sie miteinander verbunden sind, einen 3, 4, 5, 6, 7 oder 8-gliedrigen Ring, wobei ein C-Atom des Rings durch ein Sauerstoffatom oder eine NCH₃-Gruppe ersetzt sein kann;
R8 und R9 unabhängig voneinander Wasserstoff, F, Cl oder CH₃;
R10 Wasserstoff oder Methyl;
sowie deren pharmazeutisch verträgliche Salze und Trifluoracetate.

3. Verbindungen der Formel I nach Anspruch 1 und/oder 2, worin bedeuten
R1 und R3 Wasserstoff;
R2 und R4 unabhängig voneinander Wasserstoff oder Cl;
R5 Wasserstoff, Methyl oder Ethyl;
R6 Wasserstoff oder Methyl;
R7 OR12 oder NR13R14;
R12 Wasserstoff oder Alkyl mit 1, 2 oder 3 C-Atomen;
R13 und R14 unabhängig voneinander Wasserstoff, Alkyl mit 1, 2, 3, 4, 5 oder 6 C-Atomen, das teilweise oder vollständig fluoriert sein kann, Cycloalkyl mit 3, 4, 5 oder 6 C-Atomen, Phenyl, Phenylalkyl mit 1, 2 oder 3 C-Atomen im Alkylteil, Heteroaryl oder Heteroarylmethyl; wobei Heteroarylreste aromatische Ringverbindungen sind, in denen ein oder mehrere Ringatome Sauerstoffatome, Schwefelatome oder Stickstoffatome sind, bevorzugt 1, 2, 3 oder 4 Stickstoffatome, 1 oder 2 Sauerstoffatome, 1 oder 2 Schwefelatome oder eine Kombinationen aus verschiedenen Heteroatomen und wobei weiterhin die Heteroarylreste über alle Positionen angebunden sein können, wobei die Phenyl- und Heteroarylreste unsubstituiert sind oder substituiert sind mit 1, 2, 3, 4 oder 5 Resten ausgewählt aus der Gruppe bestehend aus Fluor, Chlor, Methyl, CF₃, Methoxy und OH;
wobei die Alkylreste unsubstituiert sind oder substituiert sind mit 1, 2 oder 3 Resten ausgewählt aus der Gruppe Alkoxy mit 1, 2, 3
oder 4 C-Atomen, NR15R16 und Cycloalkyl mit 3, 4, 5 oder 6 C-Atomen;
R15 und R16 unabhängig voneinander Wasserstoff oder Alkyl mit 1, 2, 3 oder 4 C-Atomen;
oder
R13 und R14 zusammen mit dem N-Atom, über das sie miteinander verbunden sind, einen 3, 4, 5, 6, 7 oder 8-gliedrigen Ring, wobei ein C-Atom des Rings durch ein Sauerstoffatom oder eine NCH₃-Gruppe ersetzt sein kann;
R8 und R9 unabhängig voneinander Wasserstoff, F, Cl oder CH₃;
R10 Wasserstoff oder Methyl;
sowie deren pharmazeutisch verträgliche Salze und Trifluoracetate.

4. Verbindungen der Formel I nach einem oder mehreren der Ansprüche 1 bis 3 ausgewählt aus der Gruppe
6,8-Dichlor-2-methyl-4-[4N-(2-ethoxy-3,4-dioxocyclobuten-1-yl)amino]phenyl-1,2,3,4-tetrahydroisochinolin,
6,8-Dichlor-2-methyl-4-[3N-(2-ethoxy-3,4-dioxocyclobuten-1-yl)amino]phenyl-1,2,3,4-tetrahydroisochinolin,
6,8-Dichlor-2-methyl-4-[2N-(2-ethoxy-3,4-dioxocyclobuten-1-yl)amino]phenyl-1,2,3,4-tetrahydroisochinolin,
6,8-Dichlor-2-methyl-4(S)-[4N-(2-ethoxy-3,4-dioxocyclobuten-1-yl)amino]phenyl-1,2,3,4-tetrahydroisochinolin,
6,8-Dichlor-2-methyl-4(S)-[3N-(2-ethoxy-3,4-dioxocyclobuten-1-yl)amino]phenyl-1,2,3,4-tetrahydroisochinolin,
6,8-Dichlor-2-methyl-4(R)-[2N-(2-ethoxy-3,4-dioxocyclobuten-1-yl)amino]phenyl-1,2,3,4-tetrahydroisochinolin,
6,8-Dichlor-2-methyl-4-[4N-(2-hydroxy-3,4-dioxocyclobuten-1-yl)amino]phenyl-1,2,3,4-tetrahydroisochinolin,
6,8-Dichlor-2-methyl-4-[3N-(2-hydroxy-3,4-dioxocyclobuten-1-yl)amino]phenyl-1,2,3,4-tetrahydroisochinolin,
6,8-Dichlor-2-methyl-4-[2N-(2-hydroxy-3,4-dioxocyclobuten-1-yl)amino]phenyl-1,2,3,4-tetrahydroisochinolin,
6,8-Dichlor-2-methyl-4-[4N-(2-amino-3,4-dioxocyclobuten-1-yl)amino]phenyl-1,2,3,4-tetrahydroisochinolin,
6,8-Dichlor-2-methyl-4-[3N-(2-amino-3,4-dioxocyclobuten-1-yl)amino]phenyl-1,2,3,4-tetrahydroisochinolin,
6,8-Dichlor-2-methyl-4-[2N-(2-amino-3,4-dioxocyclobuten-1-yl)amino]phenyl-1,2, 3,4-tetrahydroisochinolin,
6,8-Dichlor-2-methyl-4(S)-[4N-(2-amino-3,4-dioxocyclobuten-1-yl)amino]phenyl-1,2,3,4-tetrahydroisochinolin,
6,8-Dichlor-2-methyl-4(S)-[3N-(2-amino-3,4-dioxocyclobuten-1-yl)amino]phenyl-1,2,3,4-tetrahydroisochinolin,
6,8-Dichlor-2-methyl-4(R)-[2N-(2-amino-3,4-dioxocyclobuten-1-yl)amino]phenyl-1,2,3,4-tetrahydroisochinolin,
6,8-Dichlor-2-methyl-4-[4N-(2-N-cyclohexyl-N-methylamino-3,4-dioxocyclobuten-1-yl)amino]phenyl-1,2,3,4-tetrahydroisochinolin,
6,8-Dichlor-2-methyl-4-[4N-(2-dimethylamino-3,4-dioxocyclobuten-1-yl)amino]phenyl-1,2,3,4-tetrahydroisochinolin,
6,8-Dichlor-2-methyl-4-[3N-(2-dimethylamino-3,4-dioxocyclobuten-1-yl)amino]phenyl-1,2,3,4-tetrahydroisochinolin,
6,8-Dichlor-2-methyl-4(S)-[4N-(2-dimethylamino-3,4-dioxocyclobuten-1-yl)amino]phenyl-1,2,3,4-tetrahydroisochinolin,
6,8-Dichlor-2-methyl-4(R)-[2N-(2-dimethylamino-3,4-dioxocyclobuten-1-yl)amino]phenyl-1,2,3,4-tetrahydroisochinolin,
6,8-Dichlor-2-methyl-4(S)-[3N-(2-dimethylamino-3,4-dioxocyclobuten-1-yl)amino]phenyl-1,2,3,4-tetrahydroisochinolin,
6,8-Dichlor-2-methyl-4(S)-[4N-(2-N-(1-butyl)-N-methylamino-3,4-dioxocyclobuten-1-yl)amino]phenyl-1,2,3,4-tetrahydroisochinolin,
6,8-Dichlor-2-methyl-4-[4N-(2-dipropylamino-3,4-dioxocyclobuten-1-yl)amino]phenyl-1,2,3,4-tetrahydroisochinolin,
6,8-Dichlor-2-methyl-4-[4N-(2-(N-isobutyl-N-methylamino)-3,4-dioxocyclobuten-1-yl)amino]phenyl-1,2,3,4-tetrahydroisochinolin,
6,8-Dichlor-2-methyl-4-[4N-(2-(N-methoxyethyl)-N-methylamino)-3,4-dioxocyclobuten-1-yl)amino]phenyl-1,2,3,4-tetrahydroisochinolin,
6,8-Dichlor-2-methyl-4(S)-[4N-(2-diethylamino-3,4-dioxocyclobuten-1-yl)amino]phenyl-1,2,3,4-tetrahydroisochinolin,
6,8-Dichlor-2-methyl-4(S)-[4N-(2-(N-isopropyl-N-methylamino)-3,4-dioxo-cyclobuten-1-yl)amino]phenyl-1,2,3,4-tetrahydroisochinolin,
6,8-Dichlor-2-methyl-4-[4N-(2-(N-cyclopropylmethyl-N-propylamino)-3,4-dioxocyclobuten-1-yl)amino]phenyl-1,2,3,4-tetrahydroisochinolin,
6,8-Dichlor-2-methyl-4(S)-[3N-(2-diethylamino-3,4-dioxocyclobuten-1-yl)amino]phenyl-1,2,3,4-tetrahydroisochinolin,
6,8-Dichlor-2-methyl-4(S)-[3N-(2-N-methyl-N-isopropyl)amino-3,4-dioxocyclobuten-1-yl)amino]phenyl-1,2,3,4-tetrahydroisochinolin,
6,8-Dichlor-2-methyl-4(S)-[4N-(2N-ethyl-N-isopropylamino)-3,4-dioxocyclobuten-1-yl)amino]phenyl-1,2,3,4-tetrahydroisochinolin,
6,8-Dichlor-2-methyl-4(S)-[3N-(2-N-methyl-N-propylamino)-3,4-dioxocyclobuten-1-yl)amino]phenyl-1,2,3,4-tetrahydroisochinolin,
6,8-Dichlor-2-methyl-4(S)-[3N-(2-N-pyrrolidino-3,4-dioxocyclobuten-1-yl)amino]phenyl-1,2,3,4-tetrahydroisochinolin,
6,8-Dichlor-2-methyl-4(S)-[4N-(2-N-cyclopropylamino)-3,4-dioxo-cyclobuten-1-yl)amino]phenyl-1,2,3,4-tetrahydroisochinolin,
6,8-Dichlor-2-methyl-4(S)-[4N-(2-N-pyrrolidino-3,4-dioxocyclobuten-1-yl)amino]phenyl-1,2,3,4-tetrahydroisochinolin,
6,8-Dichlor-2-methyl-4(R)-[2N-(2-N-methyl-N-propylamino)-3,4-dioxocyclobuten-1-yl)amino]phenyl-1,2,3,4-tetrahydroisochinolin,
6,8-Dichlor-2-methyl-4-[4N-(2-N-benzyl-N-methylamino)-3,4-dioxo-cyclobuten-1-yl)amino]phenyl-1,2,3,4-tetrahydroisochinolin,
6,8-Dichlor-2-methyl-4(R)-[2N-(2-diethylamino-3,4-dioxocyclobuten-1-yl)amino]phenyl-1,2,3,4-tetrahydroisochinolin,
6,8-Dichlor-2-methyl-4(R)-[2N-(2-dipropylamino)-3,4-dioxocyclobuten-1-yl)amino]phenyl-1,2,3,4-tetrahydroisochinolin,
6,8-Dichlor-2-methyl-4(R)-[2N-(2-N-pyrrolidino-3,4-dioxocyclobuten-1-yl)amino]phenyl-1,2,3,4-tetrahydroisochinolin,
6,8-Dichlor-2-methyl-4(R)-[2N-(2-N-methyl-N-isopropyl)amino-3,4-dioxocyclobuten-1-yl)amino]phenyl-1,2,3,4-tetrahydroisochinolin,
6,8-Dichlor-2-methyl-4-[4N-(2-N-phenylamino-3,4-dioxocyclobuten-1-yl)amino]phenyl-1,2,3,4-tetrahydroisochinolin,
6,8-Dichlor-2-methyl-4-[2N-(2-N-methylamino-3,4-dioxocyclobuten-1-yl)amino]phenyl-1,2,3,4-tetrahydroisochinolin,
6,8-Dichlor-2-methyl-4-[2N-(2-N-(1-hexylamino)-3,4-dioxocyclobuten-1-yl)amino]phenyl-1,2,3,4-tetrahydroisochinolin,
6,8-Dichlor-2-methyl-4-[2N-(2-N-isopropylamino-3,4-dioxocyclobuten-1-yl)amino]phenyl-1,2,3,4-tetrahydroisochinolin,
6,8-Dichlor-2-methyl-4-[2N-(2-N-cyclopentylamino-3,4-dioxocyclobuten-1-yl)amino]phenyl-1,2,3,4-tetrahydroisochinolin,
6,8-Dichlor-2-methyl-4-[2N-(2N-(2-furylmethyl)amino-3,4-dioxocyclobuten-1-yl)amino]phenyl-1,2,3,4-tetrahydroisochinolin,
6,8-Dichlor-2-methyl-4-[2N-(2-N-ethylamino-3,4-dioxocyclobuten-1-yl)amino]phenyl-1,2,3,4-tetrahydroisochinolin,
6,8-Dichlor-2-methyl-4-[2N-(2-N-dimethylaminoethylamino-3,4-dioxocyclobuten-1-yl)amino]phenyl-1,2,3,4-tetrahydroisochinolin,
6,8-Dichlor-2-methyl-4-[4N-(2-N-ethylamino-3,4-dioxocyclobuten-1-yl)amino]phenyl-1,2,3,4-tetrahydroisochinolin,
6,8-Dichlor-2-methyl-4-[2N-(2N-(3-picolylmethyl)amino-3,4-dioxocyclobuten-1-yl)amino)phenyl-1,2,3,4-tetrahydroisochinolin,
6,8-Dichlor-2-methyl-4-[4N-(2N-methylamino-3,4-dioxocyclobuten-1-yl)amino]phenyl-1,2,3,4-tetrahydroisochinolin,
6,8-Dichlor-2-methyl-4-[4N-(2N-cyclopropylamino-3,4-dioxocyclobuten-1-yl)amino]phenyl-1,2,3,4-tetrahydroisochinolin,
6,8-Dichlor-2-methyl-4-[4N-(2N-isopropylamino-3,4-dioxocyclobuten-1-yl)amino]phenyl-1,2,3,4-tetrahydroisochinolin,
6,8-Dichlor-2-methyl-4-[4N-(2N-(2-furylmethyl)amino-3,4-dioxocyclobuten-1-yl)aminojphenyl-1,2,3,4-tetrahydroisochinolin,
6,8-Dichlor-2-methyl-4-[4N-(2N-dimethylaminoethylamino)-3,4-dioxocyclobuten-1-yl)amino]phenyl-1,2,3,4-tetrahydroisochinolin,
6,8-Dichlor-2-methyl-4-[4N-(1-hexylamino)-3,4-dioxocyclobuten-1-yl)amino]phenyl-1,2,3,4-tetrahydroisochinolin,
6,8-Dichlor-2-methyl-4-[4N-(3-picolylamino)-3,4-dioxo-cyclobuten-1-yl)amino]phenyl-1,2,3,4-tetrahydroisochinolin,
6,8-Dichlor-2-methyl-4-[4N-2-methylamino-3,4-dioxo-cyclobuten-1-yl)amino]phenyl-1,2,3,4-tetrahydroisochinolin,
6, 8-Dichlor-2-methyl-4-[4N-(2-ethylamino)-3,4-dioxo-cyclobuten-1-yl)amino]phenyl-1,2,3,4-tetrahydroisochinolin,
6,8-Dichlor-2-methyl-4-[3N-(2-(2-furylmethylamino)-3,4-dioxo-cyclobuten-1-yl)amino]phenyl-1,2,3,4-tetrahydroisochinolin,
6,8-Dichlor-2-methyl-4-[3N-(2-cyclopentylamino-3,4-dioxo-cyclobuten-1-yl)amino]phenyl-1,2,3,4-tetrahydroisochinolin,
6,8-Dichlor-2-methyl-4-[3N-(2-isopropylamino)-3,4-dioxo-cyclobuten-1-yl)amino]phenyl-1,2,3,4-tetrahydroisochinolin,
6,8-Dichlor-2-methyl-4-[3N-(2N-dimethylaminoethylamino)-3,4-dioxocyclobuten-1-yl)amino]phenyl-1,2,3,4-tetrahydroisochinolin,
6,8-Dichlor-2-methyl-4(R)-[2N-(2-isopropylamino-3,4-dioxocyclobuten-1-yl)amino]phenyl-1,2,3,4-tetrahydroisochinolin,
6,8-Dichlor-2-methyl-4(S)-[3N-(2-methylamino-3,4-dioxocyclobuten-1-yl)amino]phenyl-1,2,3,4-tetrahydroisochinolin
und
6,8-Dichlor-2-methyl-4(R)-[2N-(2-methylamino-3,4-dioxocyclobuten-1-yl)amino]phenyl-1,2,3,4-tetrahydroisochinolin
sowie deren pharmazeutisch verträgliche Salze und Trifluoracetate.

5. Verbindungen der Formel I und deren pharmazeutisch verträgliche Salze nach einem oder mehreren der Ansprüche 1 bis 4 zur Verwendung als Medikament.

6. Verwendung einer Verbindung der Formel I und/oder dessen pharmazeutisch verträglicher Salze nach einem oder mehreren der Ansprüche 1 bis 4 zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie-oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, von Schockzuständen oder der Zuckerkrankheit und diabetischer Spätschäden oder von Krankheiten, bei denen die Zellproliferation eine primäre oder sekundäre Ursache darstellt, zur Konservierung und Lagerung von Transplantaten für chirurgische Maßnahmen, für den Einsatz bei chirurgischen Operationen und Organtransplantationen und zur Gesunderhaltung und Lebensverlängerung.

7. Verwendung einer Verbindung der Formel I und/oder dessen pharmazeutisch verträglicher Salze nach einem oder mehreren der Ansprüche der Ansprüche 1 bis 4 in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie-oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, von Schockzuständen oder der Zuckerkrankheit und diabetischer Spätschäden oder von Krankheiten, bei denen die Zellproliferation eine primäre oder sekundäre Ursache darstellt zur Konservierung und Lagerung von Transplantaten für chirurgische Maßnahmen, für den Einsatz bei chirurgischen Operationen und Organtransplantationen und zur Gesunderhaltung und Lebensverlängerung.

8. Verwendung einer Verbindung der Formel I und/oder dessen pharmazeutisch verträglicher Salze nach einem oder mehreren der Ansprüche 1 bis 4 allein oder in Kombination mit anderen Arzneimitteln oder Wirkstoffen zur Herstellung eines Medikaments zur Behandlung oder Prophylaxe von Störungen des Atemantriebs und/oder von Schlaf-bedingten Atemstörungen wie Schlafapnoen.

9. Verwendung einer Verbindung der Formel I und/oder dessen pharmazeutisch verträglicher Salze nach einem oder mehreren der Ansprüche 1 bis 4 allein oder in Kombination mit anderen Arzneimitteln oder Wirkstoffen zur Herstellung eines Medikaments zur Behandlung oder Prophylaxe des Schnarchens.

10. Verwendung einer Verbindung der Formel I und/oder dessen pharmazeutisch verträglicher Salze nach einem oder mehreren der Ansprüche 1 bis 4 allein oder in Kombination mit anderen Arzneimitteln oder Wirkstoffen zur Herstellung eines Medikaments zur Behandlung oder zur Prophylaxe von akuten oder chronischen Nierenerkrankungen, des akuten Nierenversagens oder des chronischen Nierenversagens.

11. Verwendung einer Verbindung der Formel I und/oder dessen pharmazeutisch verträglicher Salze nach einem oder mehreren der Ansprüche 1 bis 4 allein oder in Kombination mit anderen Arzneimitteln oder Wirkstoffen zur Herstellung eines Medikaments zur Behandlung oder Prophylaxe von Störungen der Darmfunktion.

12. Heilmittel für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 4.

13. Heilmittel für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 4, in Kombination mit anderen pharmakologischen Wirkstoffen oder Arzneimitteln.

## Claims

1. A compound of the formula I 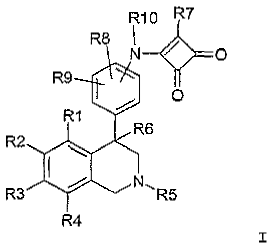 in which the meanings are:
R1, R2, R3 and R4 independently of one another hydrogen, F, Cl, Br, I, CN, NO₂ or R11-(CₘH₂ₘ)-Aₙ-;
m zero, 1, 2, 3 or 4;
n zero or 1;
R11 hydrogen, methyl, CₚF₂ₚ₊₁ or phenyl;
p 1, 2 or 3;
A oxygen, NH, N(CH₃) or S(O)_{q};
q zero, 1 or 2;
R5 hydrogen, alkyl having 1, 2, 3, 4, 5 or 6 C-atoms which may be partly or completely fluorinated, or cycloalkyl having 3, 4, 5 or 6 C-atoms;
R6 hydrogen, OH, F, CF₃, methyl, ethyl, isopropyl or cyclopropyl;
R7 hydrogen, alkyl having 1, 2, 3, 4, 5 or 6 C-atoms, cycloalkyl having 3, 4, 5 or 6 C-atoms, OR12 or NR13R14;
R12 hydrogen, alkyl having 1, 2, 3, 4, 5 or 6 C-atoms which may be partly or completely fluorinated, or cycloalkyl having 3, 4, 5 or 6 C-atoms;
R13 and R14 independently of one another hydrogen, cycloalkyl having 3, 4, 5 or 6 C-atoms; alkyl having 1, 2, 3, 4, 5 or 6 C-atoms which may be partly or completely fluorinated, phenyl, phenylalkyl having 1, 2 or 3 C-atoms in the alkyl moiety, heteroaryl or heteroarylmethyl, where heteroaryl radicals are aromatic ring compounds in which one or more ring atoms are oxygen atoms, sulfur atoms or nitrogen atoms, preferably 1, 2, 3 or 4 nitrogen atoms, 1 or 2 oxygen atoms, 1 or 2 sulfur atoms or a combination of various heteroatoms and where furthermore the heteroaryl radicals may be attached via all positions, where the phenyl and heteroaryl radicals are unsubstituted or substituted by 1, 2, 3, 4 or 5 radicals selected from the group consisting of fluorine, chlorine, methyl, CF₃, methoxy and OH;
where the alkyl radicals are unsubstituted or are substituted by 1, 2 or 3 radicals selected from the group of alkoxy having 1, 2, 3 or 4 C-atoms, NR15R16 and cycloalkyl having 3, 4, 5 or 6 C-atoms;
R15 and R16 independently of one another hydrogen or alkyl having 1, 2, 3 or 4 C-atoms;
or
R13 and R14 together with the N atom via which they are bonded together a 3, 4, 5, 6, 7, 8 or 9-membered ring, where one C-atom of the ring may be replaced by an oxygen atom or an NCH₃ group,
R8 and R9 independently of one another hydrogen, F, Cl, OH, CH₃, CH₃O, CF₃, CF₃CH₂O or CH₃SO₂;
R10 hydrogen, methyl or ethyl;
and the pharmaceutically acceptable salts and trifluoroacetates thereof.

2. A compound of the formula I as claimed in claim 1, in which the meanings are
R1, R2, R3 and R4 independently of one another hydrogen, F, Cl, Br, CN or R11-(CₘH₂ₘ)-Aₙ-;
m zero or 1;
n zero or 1;
R11 hydrogen, methyl, CₚF₂ₚ₊₁- or phenyl;
p 1 or 2;
A oxygen or S(O)_{q};
q zero, 1 or 2;
R5 hydrogen, methyl, ethyl or cyclopropyl;
R6 hydrogen or methyl;
R7 OR12 or NR13R14;
R12 hydrogen or alkyl having 1, 2 or 3 C-atoms;
R13 and R14 independently of one another hydrogen, alkyl having 1, 2, 3, 4, 5 or 6 C-atoms which may be partly or completely fluorinated, cycloalkyl having 3, 4, 5 or 6 C-atoms, phenyl, phenylalkyl having 1, 2 or 3 C-atoms in the alkyl moiety, heteroaryl or heteroarylmethyl, where heteroaryl radicals are aromatic ring compounds in which one or more ring atoms are oxygen atoms, sulfur atoms or nitrogen atoms, preferably 1, 2, 3 or 4 nitrogen atoms, 1 or 2 oxygen atoms, 1 or 2 sulfur atoms or a combination of various heteroatoms and where furthermore the heteroaryl radicals may be attached via all positions,
where the phenyl and heteroaryl radicals are unsubstituted or are substituted by 1, 2, 3, 4 or 5 radicals selected from the group consisting of fluorine, chlorine, methyl, CF₃, methoxy and OH;
where the alkyl radicals are unsubstituted or are substituted by 1, 2 or 3 radicals selected from the group of alkoxy having 1, 2, 3 or 4 C-atoms, NR15R16 and cycloalkyl having 3, 4, 5 or 6 C-atoms;
R15 and R16 independently of one another hydrogen or alkyl having 1, 2, 3 or 4 C-atoms;
or
R13 and R14 together with the N atom via which they are bonded together a 3, 4, 5, 6, 7 or 8-membered ring, where one C-atom of the ring may be replaced by an oxygen atom or an NCH₃ group;
R8 and R9 independently of one another hydrogen, F, Cl or CH3;
R10 hydrogen or methyl;
and the pharmaceutically acceptable salts and trifluoroacetates thereof.

3. A compound of the formula I as claimed in claim 1 and/or 2, in which the meanings are
R1 and R3 hydrogen;
R2 and R4 independently of one another hydrogen or Cl;
R5 hydrogen, methyl or ethyl;
R6 hydrogen or methyl;
R7 OR12 or NR13R14;
R12 hydrogen or alkyl having 1, 2 or 3 C-atoms;
R13 and R14 independently of one another hydrogen, alkyl having 1, 2, 3, 4, 5 or 6 C-atoms which may be partly or completely fluorinated, cycloalkyl having 3, 4, 5 or 6 C-atoms, phenyl, phenylalkyl having 1, 2 or 3 C-atoms in the alkyl moiety, heteroaryl or heteroarylmethyl; where heteroaryl radicals are aromatic ring compounds in which one or more ring atoms are oxygen atoms, sulfur atoms or nitrogen atoms, preferably 1, 2, 3 or 4 nitrogen atoms, 1 or 2 oxygen atoms, 1 or 2 sulfur atoms or a combination of various heteroatoms and where furthermore the heteroaryl radicals may be attached via all positions, where the phenyl and heteroaryl radicals are unsubstituted or are substituted by 1, 2, 3, 4 or 5 radicals selected from the group consisting of fluorine, chlorine, methyl, CF3, methoxy and OH; where the alkyl radicals are unsubstituted or are substituted by 1, 2 or 3 radicals selected from the group of alkoxy having 1, 2, 3 or 4 C-atoms, NR15R16 and cycloalkyl having 3, 4, 5 or 6 C-atoms;
R15 and R16 independently of one another hydrogen or alkyl having 1, 2, 3 or 4 C-atoms;
or
R13 and R14 together with the N atom via which they are bonded together a 3, 4, 5, 6, 7 or 8-membered ring, where one C-atom of the ring may be replaced by an oxygen atom or an NCH₃ group;
R8 and R9 independently of one another hydrogen, F, Cl or CH₃;
R10 hydrogen or methyl;
and the pharmaceutically acceptable salts and trifluoroacetates thereof.

4. A compound of the formula I as claimed in one or more of claims 1 to 3 selected from the group
6,8-dichloro-2-methyl-4-[4N-(2-ethoxy-3,4-dioxocyclobuten-1-yl)amino]phenyl-1,2,3,4-tetrahydroisoquinoline,
6,8-dichloro-2-methyl-4-[3N-(2-ethoxy-3,4-dioxocyclobuten-1-yl)amino]phenyl-1,2,3,4-tetrahydroisoquinoline,
6,8-dichloro-2-methyl-4-[2N-(2-ethoxy-3,4-dioxocyclobuten-1-yl)amino]phenyl-1,2,3,4-tetrahydroisoquinoline,
6,8-dichloro-2-methyl-4(S)-[4N-(2-ethoxy-3,4-dioxocyclobuten-1-yl)amino]phenyl-1,2,3,4-tetrahydroisoquinoline,
6,8-dichloro-2-methyl-4(S)-[3N-(2-ethoxy-3,4-dioxocyclobuten-1-yl)amino]phenyl-1,2,3,4-tetrahydroisoquinoline,
6,8-dichloro-2-methyl-4(R)-[2N-(2-ethoxy-3,4-dioxocyclobuten-1-yl)amino]phenyl-1,2,3,4-tetrahydroisoquinoline,
6,8-dichloro-2-methyl-4-[4N-(2-hydroxy-3,4-dioxocyclobuten-1-yl)amino]phenyl-1,2,3,4-tetrahydroisoquinoline,
6,8-dichloro-2-methyl-4-[3N-(2-hydroxy-3,4-dioxocyclobuten-1-yl)amino]phenyl-1,2,3,4-tetrahydroisoquinoline,
5,8-dichloro-2-methyl-4-[2N-(2-hydroxy-3,9-dioxocyclobuten-1-yl)amino]phanyl-1,2,3,4-tetrahydroisoquinoline,
6,8-dichloro-2-methyl-4-[4N-(2-amino-3,4-dioxocyclobuten-1-yl)amino]phenyl-1,2,3,4-tetrahydroisoquinoline,
6,8-dichloro-2-methyl-4-[3N-(2-amino-3,4-dioxocyclobuten-1-yl)amino]phenyl-1,2,3,4-tetrahydroisoquinoline,
6,8-dichloro-2-methyl-4-[2N-(2-amino-3,4-dioxocyclobuten-1-yl)amino]phenyl-1,2,3,4-tetrahydroisoquinoline,
6,8-dichloro-2-methyl-4(S)-[4N-(2-amino-3,4-dioxocyclobuten-1-yl)amino]phenyl-1,2,3,4-tetrahydroisoquinoline,
6,8-dichloro-2-methyl-4(S)-[3N-(2-amino-3,4-dioxocyclobuten-1-yl)amino]phenyl-1,2,3,4-tetrahydroisoquinoline,
6,8-dichloro-2-methyl-4(R)-[2N-(2-amino-3,4-dioxocyclobuten-1-yl)amino]phenyl-1,2,3,4-tetrahydroisoquinoline,
6,8-dichloro-2-methyl-4-[4N-(2-N-cyclohexyl-N-methylamino-3,4-dioxocyclobuten-1-yl)amino]phenyl-1,2,3,4-tetrahydroisoquinoline,
6,8-dichloro-2-methyl-4-[4N-(2-dimethylamino-3,4-dioxocyclobuten-1-yl)-amino]phenyl-1,2,3,4-tetrahydroisoquinoline,
6,8-dichloro-2-methyl-4-[3N-(2-dimethylamino-3,4-dioxocyclobuten-1-yl)amino]phenyl-1,2,3,4-tetrahydroisoquinoline,
6,8-dichloro-2-methyl-4(S)-[4N-(2-dimethylamino-3,4-dioxocyclobuten-1-yl)amino]phenyl-1,2,3,4-tetrahydroisoquinoline,
6,8-dichloro-2-methyl-4(R)-[2N-(2-dimethylamino-3,4-dioxocyclobuten-1-yl)amino]phenyl-1,2,3,4-tetrahydroisoquinoline,
6,8-dichloro-2-methyl-4(S)-[3N-(2-dimethylamino-3,4-dioxocyclobuten-1-yl)aminolphenyl-1,2,3,4-tetrahydroisoquinoline,
6,8-dichloro-2-methyl-4(S)-[4N-(2-N-(1-butyl)-N-methylamino-3,4-dioxocyclobuten-1-yl)amino]phenyl-1,2,3,4-tetrahydroisoquinoline,
6,8-dichloro-2-methyl-4-[4N-(2-dipropylamino-3,4-dioxocyclobuten-1-yl)amino]phenyl-1,2,3,4-tetrahydroisoquinoline,
6,8-dichloro-2-methyl-4-[4N-(2-(N-isobutyl-N-methylamino)-3,4-dioxocyclobuten-1-yl)amino]phenyl-1,2,3,4-tetrahydroisoquinoline,
6,8-dichloro-2-methyl-4-[4N-(2-(N-methoxyethyl)-N-methylamino)-3,4-dioxocyclobuten-1-yl)amino]phenyl-1,2,3,4-tetrahydroisoquinoline,
5,8-dichloro-2-methyl-4(S)-[4N-(2-diethylamino-3,4-dioxocyclobuten-2-yl)amino]phenyl-1,2,3,a-tetrahydroisoquinoline,
6,8-dichloro-2-methyl-4(S)-[4N-(2-(N-isopropyl-N-methylamino)-3,4-dioxocyclobuten-1-yl)amino]phenyl-1,2,3,4-tetrahydroisoquinoline,
6,8-dichloro-2-methyl-4-[4N-(2-(N-cyclopropylmethyl-N-propylamino)-3,4-dioxocyclobuten-1-y1)amino]phenyl-1,2,3,4-tetrahydroisoquinoline,
6,8-dichloro-2-methyl-4(S)-[3N-(2-diethylamino-3,4-dioxocyclobuten-1-yl)amino]phenyl-1,2,3,4-tetrahydroisoquinoline,
6,8-dichloro-2-methyl-4(S)-[3N-(2-N-methyl-N-isopropyl)amino-3,4-dioxocyclobuten-1-yl)amino]phenyl-1,2,3,4-tetrahydroisoquinoline,
6,8-dichloro-2-methyl-4(S)-[4N-(2N-ethyl-N-isopropyl)amino-3,4-dioxocyclobuten-1-yl)amino]phenyl-1,2,3,4-tetrahydroisoquinoline,
6,8-dichloro-2-methyl-4(S)-[3N-(2-N-methyl-N-propyl)amino-3,4-dioxocyclobuten-1-yl)amino]phenyl-1,2,3,4-tetrahydroisoquinoline,
6,8-dichloro-2-methyl-4(S)-[3N-(2-N-pyrrolidino-3,4-dioxocyclobuten-1-yl)amino7phenyl-1,2,3,4-tetrahydroisoquinoline,
6,8-dichloro-2-methyl-4(S)-[4N-(2-N-cyclopropylamino)-3,4-dioxocyclobuten-1-yl)amino]phenyl-1,2,3,4-tetrahydroisoquinoline,
6,8-dichloro-2-methyl-4(S)-[4N-(2-N-pyrrolidino-3,4-dioxocyclobuten-1-yl)amino]phenyl-1,2,3,4-tetrahydroisoquinoline,
6,8-dichloro-2-methyl-4(R)-[2N-(2-N-methyl-N-propylamino)-3,4-dioxocyclobuten-1-yl)amino]phenyl-1,2,3,4-tetrahydroisoquinoline,
6,8-dichloro-2-methyl-4-[4N-(2-N-benzyl-N-methylamino)-3,4-dioxocyclobuten-1-yl)amino]phenyl-1,2,3,4-tetrahydroisoquinoline,
6,8-dichloro-2-methyl-4(R)-[2N-(2-diethylamino-3,4-dioxocyclobuten-1-yl)amino]phenyl-1,2,3,4-tetrahydroisoquinoline,
6,8-dzchloro-2-methyl-4(R)-[2N-(2-dipropylamino)-3,4-dioxocyclobuten-1-yl)amino]phenyl-1,2,3,4-tetrahydroisoquinoline,
6,8-dichloro-2-methyl-4(R)-[2N-(2-N-pyrrolidino-3,4-dioxocyclobuten-1-yl)amino]phenyl-1,2,3,4-tetrahydroisoquinoline,
6,8-dichloro-2-methyl-4(R)-[2N-(2-N-methyl-N-isopropyl)amino-3,4-dioxocyclobuten-1-yl)amino]phenyl-1,2,3,4-tetrahydroisoquinoline,
6,8-dichloro-2-methyl-4-[4N-(2-N-phenylamino-3,4-dioxocyclobuten-1-yl)amino]phenyl-1,2,3,4-tetrahydroisoquinoline,
6,8-dichloro-2-methyl-4-[2N-(2-N-methylamino-3,4-dioxocyclobuten-1-yl)amino]phenyl-1,2,3,4-tetrahydroisoquinoline,
6,8-dichloro-2-methyl-4-[2N-(2-N-(1-hexylalamino)-3,4-dioxocyclobuten-1-yl)amino]phenyl-1,2,3,4-tetrahydroisoquinoline,
6,8-dichloro-2-methyl-4-[2N-(2-N-isopropylamino-3,4-dioxocyclobuten-1-yl)amino]phenyl-1,2,3,4-tetrahydroisoquinoline,
5,8-dichloro-2-methyl-9-[2N-(2-N-cyclopentylamino-3,4-dioxocyclobuten-1-yl)amino]phenyl-1,2,3,4-tetrahydroisoquinoline,
6,8-dichloro-2-methyl-4-[2N-(2N-(2-furylmethyl)amino-3,4-dioxocyclobuten-1-yl)amino]phenyl-1,2,3,4-tetrahydroisoquinoline,
6,8-dichloro-2-methyl-4-[2N-(2-N-ethylamino-3,4-dioxocyclobuten-1-yl)amino]phenyl-1,2,3,4-tetrahydroisoquinoline,
6,8-dichloro-2-methyl-4-[2N-(2-N-dimethylaminoethylamino-3,4-dioxocyclobuten-l-yl)amino]phenyl-1,2,3,4-tetrahydroisoquinoline,
6,8-dichloro-2-methyl-4-[4N-(2-N-ethylamino-3,4-dioxocyclobuten-1-yl)amino]phenyl-1,2,3,4-tetrahydroisoquinoline,
6,8-dichloro-2-methyl-4-[2N-(2N-(3-picolylmethyl)amino-3,4-dioxocyclobuten-1-yl)aminolphenyl-1,2,3,4-tetrahydroisoquinoline,
6,8-dichloro-2-methyl-4-[4N-(2N-methylamino-3,4-dioxocyclobuten-1-yl)amino]phenyl-1,2,3,-tetrahydroisoquinoline,
6,8-dichloro-2-methyl-4-[4N-(2N-cyclopropylamino-3,4-dioxocyclebuten-1-yl)amino]phenyl-1,2,3,4-tetrahydroisoquinoline,
6,9-dichloro-2-methyl-4-[4N-(2N-isopropylamino-3,4-dioxocyclobuten-1-yl)amino]phenyl-1,2,3,4-tetrahydroisoquinoline,
6,8-dichloro-2-methyl-4-[4N-(2N-(2-furylmethyl)amine-3,4-dioxocyclobuten-1-yl)amiriolphenyl-1,2,3,4-tetrahydroisoquinoline,
5,9-dichloro-2-methyl-4-[4N-(2N-dimethylaminoethylamino)-3,4-dioxocyclobuten-1-yl)amino]phenyl-1,2,3,4-tetrahydroisoquinoline,
6,8-dichloro-2-methyl-4-[4N-(1-hexylamino)-3,4-dioxocyclobuten-1-yl)amino]phenyl-1,2,3,4-tetrahydroisoquinoline,
6,8-dichloro-2-methyl-4-[4N-(3-picolylamino)-3,4-dioxocyclobuten-1-yl)aminolphenyl-1,2,3,4-tetrahydroisoquinoline,
6,9-dichloro-2-methyl-4-[4N-2-methylamino-3,4-dioxocyclobuten-1-yl)amino]phenyl-2,2,3,4-tetrahydroisoquinoline,
6,9-dichloro-2-methyl-4-[4N-(2-ethylamino)-3,4-dioxocyclobuten-1-yl)amino]phenyl-1,2,3,4-tetrahydroisoquinoline,
6,8-dichloro-2-methyl-4-[3N-(2-(2-furylmethylamino)-3,4-dioxocyclobuten-1-yl)amino]phenyl-1,2,3,4-tetrahydroisoquinoline,
6,8-dichloro-2-methyl-4-[3N-(2-cyclopentylamino-3,4-dioxocyclobuten-1-yl)amino]phenyl-1,2,3,4-tetrahydroisoquinoline,
6,8-dichloro-2-methyl-4-[3N-(2-isopropylamino)-3,4-dioxocyclobuten-1-yl)amino]phenyl-1,2,3,4-tetrahydroisoquinoline,
6,8-dichloro-2-methyl-4-[3N-(2N-dimethylaminoethylamino)-3,4-dioxocyclobuten-1-yl)amino]phenyl-1,2,3,4-tetrahydroisoquinoline,
6,8-dichloro-2-methyl-4(R)-[2N-(2-isopropylamino-3,4-dioxocyclobuten-1-yl)amino]phenyl-1,2,3,4-tetrahydroisoquinoline,
6,8-dichloro-2-methyl-4(S)-[3N-(2-methylamino-3,4-dioxocyclobuten-1-yl)amino]phenyl-1,2,3,4-tetrahydroisoquinoline
and
6,8-dichloro-2-methyl-4(R)-[2N-(2-methylamino-3,4-dioxocyclobuten-1-yl)amino]phenyl-1,2,3,4-tetrahydroisoquinoline
and the pharmaceutically acceptable salts and trifluoroacetates thereof.

5. A compound of the formula I and the pharmaceutically acceptable salts thereof as claimed in one or more of claims 1 to 4 for use as medicament.

6. The use of a compound of the formula I and/or its pharmaceutically acceptable salts as claimed in one or more of claims 1 to 4 for the manufacture of a medicament for the treatment or prophylaxis of disorders of respiratory drive, of respiratory disorders, sleep-related respiratory disorders, sleep apneas, of snoring, of acute and chronic renal disorders, of acute renal failure and of chronic renal failure, of disorders of bowel function, of high blood pressuxre, of essential hypertension, diseases of central nervous system, of diseases resulting from CNS overexcitability, epilepsy and centrally induced spasms or of anxiety states, depressions and psychoses, of ischemic states of the peripheral or central nervous system or of stroke, of acute and chronic damage and disorders of peripheral organs or limbs caused by ischemic or reperfusion events, of atherosclerosis, of disorders of lipid metabolism, of thromboses, of disorders of biliary function, of infestation by ectoparasites, of diseases resulting from endothelial dysfunction, of protozoal diseases, of malaria, of states of shock or of diabetes and late damage from diabetes or of diseases in which cell proliferation represents a primary or secondary cause, for the preservation and storage of transplants for surgical procedures, for use in surgical operations and organ transplantations and for maintaining health and prolonging life.

7. The use of a compound of the formula I and/or its pharmaceutically acceptable salts as claimed in one or more of claims 1 to 4 in combination with other pharmaceuticals or active ingredients for the manufacture of a medicament for the treatment or prophylaxis of disorders of respiratory drive, of respiratory disorders, sleep-related respiratory disorders, sleep apneas, of snoring, of acute and chronic renal disorders, of acute renal failure and of chronic renal failure, of disorders of bowel function, of high blood pressure, of essential hypertension, diseases of central nervous system, of disorders resulting from CNS overexcitability, epilepsy and centrally induced spasms or of anxiety states, depressions and psychoses, of ischemic states of the peripheral or central nervous system or of stroke, of acute and chronic damage and disorders of peripheral organs or limbs caused by ischemic or reperfusion events, of atherosclerosis, of disorders of lipid metabolism, of thromboses, of disorders of biliary function, of infestation by ectoparasites, of diseases resulting from endothelial dysfunction, of protozoal diseases, of malaria, of states of shock or of diabetes and late damage from diabetes or of diseases in which cell proliferation represents a primary or secondary cause, for the preservation and storage of transplants for surgical procedures, for use in surgical operations and organ transplantations and for maintaining health and prolonging life.

8. The use of a compound of the formula I and/or its pharmaceutically acceptable salts as claimed in one or more of claims 1 to alone or in combination with other pharmaceuticals or active ingredients for the manufacture of a medicament for the treatment or prophylaxis of disorders of the respiratory drive and/or of sleep-related respiratory disorders such as sleep apneas.

9. The use of a compound of the formula I and/or its pharmaceutically acceptable salts as claimed in one or more of claims 1 to 4 alone or in combination with other pharmaceuticals or active ingredients for the manufacture of a medicament for the treatment or prophylaxis of snoring.

10. The use of a compound of the formula I and/or its pharmaceutically acceptable salts as claimed in one or more of claims 1 to 4 alone or in combination with other pharmaceuticals or active ingredients for the manufacture of a medicament for the treatment or prophylaxis of acute or chronic renal disorders, of acute renal failure or of chronic renal failure.

11. The use of a compound of the formula I and/or its pharmaceutically acceptable salts as claimed in one or more of claims 1 to 4 alone or in combination with other pharmaceuticals or active ingredients for the manufacture of a medicament for the treatment or prophylaxis of disorders of bowel function.

12. A medicine for human, veterinary or phytoprotective use comprising an effective amount of a compound of the formula I and/or of a pharmaceutically acceptable salt thereof as claimed in one or more of claims 1 to 4.

13. A medicine for human, veterinary or phytoprotective use comprising an effective amount of a compound of the formula I and/or of a pharmaceutically acceptable salt thereof as claimed in one or more of claims 1 to 4, in combination with other pharmacological active ingredients or pharmaceuticals.

## Revendications

1. Composés de fox-mule I 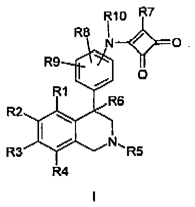 dans laquelle :
R¹, R², R³ et R⁴ signifient, indépendamment l'un de l'autre, hydrogène, F, Cl, Br, 1, CN, NO₂ au R¹¹-(CₘH₂ₘ)-Aₙ- ;
m vaut zéro, 1, 2, 3 ou 4 ;
n vaut zéro ou 1 ;
R¹¹ signifie hydrogène, méthyle, CₚF₂ₚ₊₁ ou phényle ;
p vaut 1, 2 ou 3 ;
A signifie oxygène, NH, N(CH₃) ou S(O)q ;
q vaut zéro, 1 ou 2 ;
R⁵ signifie hydrogène, alkyle comprenant 1, 2, 3, 4, 5 ou 6 atomes de carbone, qui peut être partiellement ou totalement fluoré, ou cycloalkyle comprenant 3, 4, 5 ou 6 atomes de carbone ;
R⁶ signifie hydrogène, OH, F, CF₃, méthyle, éthyle, isopropyle ou cyclopropyle ;
R⁷ signifie hydrogène, alkyle comprenant 1, 2, 3, 4, 5 ou 6 atomes de carbone, cycloalkyle comprenant 3, 4, 5 ou 6 atomes de carbone, OR¹² ou NR¹³R¹⁴ ;
R¹² signifie hydrogène, alkyle comprenant 1, 2, 3, 4, 5 ou 6 atomes de carbone, qui peut être partiellement ou totalement fluoré, ou cycloalkyle comprenant 3, 4, 5 ou 6 atomes de carbone ;
R¹³ et R¹⁴ signifient, indépendamment l'un de l'autre, hydrogène, cycloalkyle comprenant 3, 4, 5 ou 6 atomes de carbone, alkyle comprenant 1, 2, 3, 4, 5 ou 6 atomes de carbone, qui peut être partiellement ou complètement fluoré, phényle, phénylalkyle comprenant 1, 2 ou 3 atomes de carbone dans la partie alkyle, hétéroaryle ou hétéroarylméthyle, les radicaux hétéroaryle étant des composés cycliques aromatiques dans lesquels un ou plusieurs atomes de cycle sont des atomes d'oxygène, de soufre ou d'azote, de préférence 1, 2, 3 ou 4 atomes d'azote, 1 ou 2 atomes d'oxygène, 1 ou 2 atomes de soufre ou une combinaison de différents hétéroatomes et les radicaux hétéroaryle pouvant en outre être liés via toutes les positions, les radicaux phényle et hétéroaryle étant non substitués ou substitués par 1, 2, 3, 4 ou 5 radicaux choisis dans le groupe constitué par fluor, chlore, méthyle, CF₃, méthoxy et OH ;
les radicaux alkyle étant non substitués ou substitués par 1, 2 ou 3 radicaux choisis dans le groupe constitué par alcoxy comprenant 1, 2, 3 ou 4 atomes de carbone, NR¹⁵R¹⁶ et cycloalkyle comprenant 3, 4, 5 ou 6 atomes de carbone ;
R¹⁵ et R¹⁶ signifient, indépendamment l'un de l'autre, hydrogène ou alkyle comprenant 1, 2, 3 au 4 atomes de carbone ; ou
R¹³ et R¹⁴ signifient, ensemble avec l'atome de N via lequel ils sont liés l'un à l'autre, un cycle de 3, 4, 5, 6, 7, 8 ou 9 chaînons, un atome de C du cycle pouvant être remplacé par un atome d'oxygène ou un groupe NCH₃,
R⁸ et R⁹ signifient, indépendamment l'un de l'autre, hydrogène, F, Cl, OH, CH₃, CH₃O, CF₃, CF₃CH₂O ou CH₃SO₂ ;
R¹⁰ signifie hydrogène, méthyle ou éthyle ;
ainsi que leurs sels pharmaceutiquement acceptables et leurs trifluoroacétates.

2. Composés de formule 1 selon la revendication 1, dans laquelle :
R¹, R², R³ et R⁴ signifient, indépendamment l'un de l'autre, hydrogène, F, Cl, Br, CN, R¹¹-(CₘH₂ₘ)-An- ;
m vaut zéro ou 1 ;
n vaut zéro ou 1 ;
R¹¹ signifie hydrogène, méthyle, CpF₂ₚ₊₁ ou phényle ;
p vaut 1 ou 2 ;
A signifie oxygène ou S(O)_{q} ;
q vaut zéro, 1 ou 2 ;
R⁵ signifie hydrogène, méthyle, éthyle ou cyclopropyle ;
R⁶ signifie hydrogène ou méthyle ;
R⁷ signifie OR¹² ou NR¹³R¹⁴ ;
R¹² signifie hydrogène ou alkyle comprenant 1, 2 ou 3 atomes de carbone ;
R¹³ et R¹⁴ signifient, indépendamment l'un de l'autre, hydrogène, alkyle comprenant 1, 2, 3, 4, 5 ou 6 atomes de carbone qui peut être partiellement ou complètement fluoré, cycloalkyle comprenant 3, 4, 5 ou 6 atomes de carbone, phényle, phénylalkyle comprenant 1, 2 ou 3 atomes de carbone dans la partie alkyle, hétéroaryle ou hétéroarylméthyle ; les radicaux hétéroaryle étant des composés cycliques aromatiques dans lesquels un ou plusieurs atomes de cycle sont des atomes d'oxygène, de soufre ou d'azote, de préférence 1, 2, 3 ou 4 atomes d'azote, 1 ou 2 atomes d'oxygène, 1 ou 2 atomes de soufre ou une combinaison de différents hétéroatomes et les radicaux hétéroaryle pouvant être liés via toutes les positions, les radicaux phényle et hétéroaryle étant non substitués ou substitués par 1, 2, 3, 4 ou 5 radicaux choisis dans le groupe constitué par fluor, chlore, méthyle, CF₃, méthoxy et OH ;
les radicaux alkyle étant non substitués ou substitués par 1, 2 ou 3 radicaux choisis dans le groupe constitué par alcoxy comprenant 1, 2, 3 ou 4 atomes de carbone, NR¹⁵R¹⁶ et cycloalkyle comprenant 3, 4, 5 ou 6 atomes de carbone ;
R¹⁵ et R¹⁶ signifient, indépendamment l'un de l'autre, hydrogène ou alkyle comprenant 1, 2, 3 ou 4 atomes de carbone ; ou
R¹³ et R¹⁴ signifient, ensemble avec l'atome de N via lequel ils sont liés l'un à l'autre, un cycle de 3, 4, 5, 6, 7 ou 8 chaînons, un atome de C du cycle pouvant être remplacé par un atome d'oxygène ou un groupe NCH₃ ;
R⁸ et R⁹ signifient, indépendamment l'un de l'autre hydrogène, F, Cl ou CH₃ ;
R¹⁰ signifie hydrogène ou méthyle ;
ainsi que leurs sels pharmaceutiquement acceptables et leurs trifluoroacétates.

3. Composés de formule 1 selon la revendication 1 et/ou 2, où
R¹ et R³ signifie hydrogène ;
R² et R⁴ signifient, indépendamment l'un de l'autre hydrogène ou Cl ;
R⁵ signifie hydrogène, méthyle ou éthyle ;
R⁶ signifie hydrogène ou méthyle ;
R⁷ signifie OR¹² ou NR¹³R¹⁴ ;
R¹² signifie hydrogène ou alkyle comprenant 1, 2 ou 3 atomes de carbone ;
R¹³ et R¹⁴ signifient, indépendamment l'un de l'autre, hydrogène, alkyle comprenant 1, 2, 3, 4, 5 ou 6 atomes de carbone qui peut être partiellement ou complètement fluoré, cjrcloalkyle comprenant 3, 4, 5 ou 6 atomes de carbone, phényle, phénylalkyle comprenant 1, 2 ou 3 atomes de carbone dans la partie alkyle, hétéroaryle ou hétéroarylméthyle ; les radicaux hétéroaryle étant des composés cycliques aromatiques dans lesquels un ou plusieurs atomes de cycle sont des atomes d'oxygène, de soufre ou d'azote, de préférence 1, 2, 3 ou 4 atomes d'azote, 1 ou 2 atomes d'oxygène, 1 ou 2 atomes de soufre ou une combinaison de différents hétéroatomes et les radicaux hétéroaryle pouvant être liés via toutes les positions, les radicaux phényle et hétéroaryle étant non substitués ou substitués par 1, 2, 3, 4 ou 5 radicaux choisis dans le groupe constitué par fluor, chlore, méthyle, CF₃, méthoxy et OH ;
les radicaux alkyle étant non substitués ou substitués par 1, 2 ou 3 radicaux choisis dans le groupe constitué par alcoxy comprenant 1, 2, 3 ou 4 atomes de carbone, NR¹⁵R¹⁶ et cycloalkyle comprenant 3, 4, 5 ou 6 atomes de carbone ;
R¹⁵ et R¹⁶ signifient, indépendamment l'un de l'autre, hydrogène ou alkyle comprenant 1, 2, 3 ou 4 atomes de carbone ; ou
R¹³ et R¹⁴ signifient, ensemble avec l'atome de N via lequel ils sont liés l'un à l'autre, un cycle de 3, 4, 5, 6, 7 ou 8 chaînons, un atome de C du cycle pouvant être remplacé par un atome d'oxygène ou un groupe NCH₃ ;
R⁸ et R⁹ signifient, indépendamment l'un de l'autre hydrogène, F, Cl ou CH₃ ;
R¹⁰ signifie hydrogène ou méthyle ;
ainsi que leurs sels pharmaceutiquement acceptables et leurs trifluoroacétates.

4. Composés de formule 1 selon l'une ou plusieurs des revendications 1 à 3, choisis dans le groupe formé par
la 6,8-dichloro-2-méthyl-4-[4N-(2-éthoxy-3,4-dioxocyclobutène-1-yl)amino]phényl-1,2,3,4-tétrahydro-isoquinoléine,
la 6,8-dichloro-2-méthyl-4-[3N-(2-éthoxy-3,4-dioxocyclobutène-1-yl)aminolphényl-1,2,3,4-tétrahydro-isoquinoléine,
la 6,8-dichloro-2-mêthyl-4-[2N-(2-ëthpxy-3,4-dioxocyclobutène-1-yl)amino]phényl-1,2,3,4-tétrahydro-isoquinoléine,
la 6,8-dichloro-2-mèthyl-4(S)-[4N-(2-éthoxy-3,4-dioxocyclobutène-1-yl)amino]phényl-1,2,3,-tétrahydro-isoquinoléine,
la 6,8-dichloro-2-méthyl-4(S)-[3N-(2-éthoxy-3,4-dioxocyclobutène-1-yl)aminolphényl-1,2,3,4-tétrahydro-isoquinoléine,
la 6,8-dichloro-2-méthyl-4(R)-[2N-(2-éthoxy-3,4-dioxocyclobutène-1-yl)amino]phényl-1,2,3,4-tétrahydro-isoquinoléine,
la 6,8-dichloro-2-méthyl-4-[4N-(2-hydroxy-3,4-dioxocyclobuténe-1-yl)amino]phényl-1,2,3,4-tétrahydro-isoquinoléine,
la 6,8-dichloro-2-méthyl-4-[3N-(2-hydroxy-3,4-dioxocyclobuténe-1-yl)amino]phényl-1,2,3,4-tétrahydro-isoquinoléine,
la 6,8-dichloro-2-méthyl-4-[2N-(2-hydroxy-3,4-dioxocyclobutène-1-yl)amino]phényl-1,2,3,4-tétrahydro-isoquinoléine,
la 6,9-dichloro-2-mêthyl-4-[4N-(2-amsno-3,4-dioxocyclobutène-1-yl)amino]phényl-1,2,3,4-tétrahydro-isoquinoléine,
la 5,8-dichloro-2-méthyl-4-[3N-(2-amino-3,4-dioxocyclobutène-1-yl)amino]phényl-1,2,3,-tétrahydro-isoquinoléine,
la 6,8-dichloro-2-méthyl-4-[2N-(2-amino-3,4-dioxocyclobutène-1-yl)amino]phényl-1,2,3,4-tétrahydro-isoquinoléine,
la 6,8-dichloro-2-méthyl-4(S)-[4N-(2-amino-3,4-dioxocyclobuténe-1-yl)amino]phényl-1,2,3,-tétrahydro-isoquinoléine,
la 6,8-dichloro-2-méthyl-4(S)-[3N-(2-amino-3,4-dioxocyclobuténe-1-yl)amino]phényl-1,2,3,4-tétrahydro-isoquinoléine,
la 6,8-dichloro-2-méthyl-4(R)-[2N-(2-amino-3,4-dioxocyclobutène-1-yl)amino]phényl-1,2,3,4-tétrahydro-isoquinoléine,
la 6,8-dichloro-2-méthyl-4-[4N-(2-N-cyclohexyl-N-méthylamino-3,4-dioxocyclobuténe-1-yl)amino]phényl-1,2,3,4-tétrahydro-isoquinoléine,
la 6,8-dichloro-2-méthyl-4-[4N-(2-diméthylamino-3,4-dioxocyclobuténe-1-yl)amino]phényl-1,2,3,4-tétrahydro-isoquinoléine,
la 6,8-dichloro-2-méthyl-4-[3N-(2-diméthylamino-3,4-dioxocyclobutène-1-yl)amino]phényl-1,2,3,4-tétrahydro-isoquinoléine,
la 6,8-dichloro-2-méthyl-4(S)-[4N-(2-diméthylamino-3,4-dioxocyclobutène-1-yl)amino]phényl-1,2,3,4-tétrahydro-isoquinoléine,
la 6,8-dichloro-2-méthyl-4(R)-[2N-(2-diméthylamino-3,4-dioxocyclobuténe-1-yl)amino]phényl-1,2,3,4-tétrahydro-isoquinoléine,
la 6,8-dichloro-2-méthyl-4(S)-[3N-(2-diméthylamino-3,4-dioxocyclobutène-1-yl)amino]phényl-1,2,3,4-tétrahydro-isoquinoléine,
à 6,8-dichloro-2-méthyl-4(S)-[4N-(2-N-(1-butyl)-N-méthylamino-3,4-dioxocyclobutène-1-yl)amino]phényl-1,2,3,4-tétrahydro-isoquinoléine,
la 6,8-dichloro-2-méthyl-4-[4N-(2-dipropylamino-3,4-dioxocyclobutène-1-yl)amino]phényl-1,2,3,4-tétrahydro-isoquinoléine,
la 6,8-dichloro-2-méthyl-4-[4N-(2-(N-isobutyl-N-méthylamino)-3,4-dioxocyclobutène-1-yl)amino]phényl-1,2,3,4-tétrahydro-isoquinoléine,
la 6,8-dichloro-2-méthyl-4-[4N-(2-(N-méthoxyéthyl)-N-méthylamino)-3,4-dioxocyclobutène-1-yl)amino]phényl-1,2,3,4-tétrahydro-isoquinoléine,
la 6,8-dichloro-2-méthyl-4(S)-[4N-(2-diéthylamino-3,4-dioxocyclobutène-1-yl)amino]phényl-1,2,3,4-tétrahydro-isoquinoléine,
la 6,8-dichloro-2-méthyl-4(S)-[4N-(2-(N-isopropyl-N-méthylamino)-3,4-dioxocyclobutène-1-yl)amino]phényl-1,2,3,4-tétrahydro-isoquinoléine,
la 6,8-dichloro-2-méthyl-4-[4N-(2-(N-cyclopropylméthyl-N-propylamino)-3,4-dioxocyclobutène-1-yl)amino]phényl-1,2,3,4-tétrahydro-isoquinoléine,
la 6,8-dichloro-2-méthyl-4(S)-[3N-(2-diéthylamino-3,4-dioxocyclobutène-1-yl)aminolphényl-1,2,3,4-tétrahydro-isoquinoléine,
la 6,8-dichloro-2-méthyl-4(S)-[3N-(2-N-méthyl-N-isopropyl)amino-3,4-dioxocyclobutène-1-yl)amino]phényl-1,2,3,4-tétrahydro-isoquinoléine,
la 6,8-dichloro-2-méthyl-4(S)-[4N-(2N-éthyl-N-isopropylamino)-3,4-dioxocyclobutène-1-yl)amino]phényl-1,2,3,4-tétrahydro-isoquinoléine,
la 6,8-dichloro-2-méthyl-4(S)-[3N-(2-N-méthyl-N-propylamino)-3,4-dioxocyolobutène-1-yl)amino]phényl-1,2,3,4-tétrahydro-isoquinoléine,
la 6,8-dichloro-2-méthyl-4(S)-[3N-(2-N-pyrrolidino-3,4-dioxocyclobutène-1-yl)amino]phényl-1,2,3,4-tétrahydro-isoquinoléine,
la 6,8-dichloro-2-méthyl-4(S)-[4N-(2-N-cyclopropylamino)-3,4-dioxocyclobutène-1-yl)amino]phényl-1,2,3,4-tétrahydro-isoquinoléine,
la 6,8-dichloro-2-méthyl-4(S)-[4N-(2-N-pyrrolidino-3,4-dioxocyclobutène-1-yl)amino]phényl-1,2,3,4-tétrahydro-isoquinoléine,
la 6,8-dichloro-2-méthyl-4(R)-[2N-(2-N-méthyl-N-propylamino)-3,4-dioxocyclobutène-1-yl)amino]phényl-1,2,3,4-tétrahydro-isoquinoléine,
la 6,8-dichloro-2-méthyl-4-[4N-(2-N-benzyl-N-méthylamino)-3,4-dioxocyclobutène-1-yl)amino]phényl-1,2,3,4-tétrahydro-isoquinoléine,
la 6,8-dichloro-2-méthyl-4(R)-[2N-(2-diéthylamino-3,4-dioxocyclobutène-1-yl)amino]phényl-1,2,3,4-tétrahydro-isoquinoléine,
la 6,8-dichloro-2-méthyl-4(R)-[2N-(2-dipropylamino)-3,4-dioxocyclobutène-1-yl)amino]phényl-1,2,3,4-têtxahydro-lsoquinolêine,
la 6,8-dichloro-2-méthyl-4(R)-[2N-(2-N-pyrrolidino-3,4-dioxocyclobutène-1-yl)amino]phényl-1,2,3,4-tétrahydro-isoquinoléine,
la 6,8-dichloro-2-méthyl-4(R)-[2N-(2-N-méthyl-N-isopropyl)amino-3,4-dioxocyclobutène-1-yl)amino]phényl-1,2,3,4-tétrahydro-isoqusnolëine,
la 6,8-dichloro-2-méthyl-4-[4N-(2-N-phénylamino-3,4-dioxocyclobutène-1-yl)amino]phényl-1,2,3,4-tétrahydro-isoquinoléine,
la 6,8-dichloro-2-méthyl-4-[2N-(2-N-méthylamino-3,4-dioxocyclobuténe-1-yl) amino]phényl-1,2,3,4-tétrahydro-isoquinoléine,
la 6,8-dichloro-2-méthyl-4-[2N-(2-N-(1-hexylamino-3,4-dioxocyclobutène-1-yl)amino]phényl-1,2,3,4-tétrahydro-isoquinoléine,
la 6,8-dichloro-2-méthyl-4-[2N-(2-N-isopropylamino-3,4-dioxocyclobutène-1-yl)amino]phényl-1,2,3,4-tétrahydro-isoquinoléine,
la 6,8-dichloro-2-méthyl-4-[2N-(2-N-cyclopentylamino-3,4-dioxocyclobutène-1-yl)amino]phényl-1,2,3,4-tétrahydro-isoquinoléine,
la 6,8-dichloro-2-méthyl-4-[2N-(2N-(2-furylméthyl)amino-3,4-dioxocyclobutène-1-yl)amino]phényl-1,2,3,4-tétrahydro-isoquinoléine,
la 6,8-dichloro-2-méthyl-4-[2N-(2-N-éthylamino-3,4-dioxocyclobutène-1-yl)amino]phényl-1,2,3,4-tétrahydro-isoquinoléine,
la 6,8-dichloro-2-méthyl-4-[2N-(2-N-diméthylaminoéthylamino-3,4-dioxocyclobuténe-1-yl)amino]phényl-1,2,3,4-tétrahydro-isoquinoléine,
la 6,8-dichloro-2-méthyl-4-[4N-(2-N-éthylamino-3,4-dioxocyclobutène-1-yl)amino]phényl-1,2,3,4-tétrahydro-isoquinoléine,
la 6,8-dichloro-2-méthyl-4-[2N-(2N-(3-picolylméthyl)amino-3,4-dioxocyclobutène-1-yl)amino]phényl-1,2,3,4-tétrahydro-isoquinoléine,
la 6,8-dichloro-2-méthyl-4-[4N-(2N-méthylamino-3,4-dioxocyclobutène-1-yl)amino]phényl-1,2,3,4-tétrahydro-isoquinoléine,
la 6,8-dichloro-2-méthyl-4-[4N-[2N-cyclopropylamino-3,4-dioxocyclobutène-1-yl)amino]phényl-1,2,3,4-tétrah.ydro-isoquinolêine,
la 6,8-dichloro-2-méthyl-4-[4N-(2N-isopropylamino-3,4-dioxocyclobutène-1-yl)amino]phényl-1,2,3,4-tétrahydro-isoquinoléine,
la 6,8-dichloro-2-méthyl-4-[4N-(2N-(2-furylméthyl)amino-3,4-dioxocyclobutène-1-yl)amino]phényl-1,2,3,4-tétrahydro-isoquinoléine,
la 6,8-dichloro-2-méthyl-4-[4N-(2N-diméthylaminoéthylamino)-3,4-dioxocyclobutène-1-yl)amino]phényl-1,2,3,4-tétrahydro-isoquinoléine,
la 6,8-dichloro-2-méthyl-4-[4N-(7-hexylamino)-3,4-dioxocyclobutène-1-yl)amino]phényl-1,2,3,4-tétrahydro-isoquinoléine,
la 6,8-dichloro-2-méthyl-4-[4N-(3-picolylamino)-3,4-dioxocyclobutène-1-yl)amino]phényl-1,2,3,4-tétrahydro-isoquinoléine,
la 6,8-dichloro-2-méthyl-4-[4N-2-méthylamino-3,4-dioxocyclobutène-1-yl)amino]phényl-1,2,3,4-tétrahydro-isoquinoléine,
la 6,8-dichloro-2-méthyl-4-[4N-(2-éthylamino)-3,4-dioxocyclobutène-1-yl)amino]phényl-1,2,3,4-tétrahydro-isoquinoléine,
la 6,8-dichloro-2-méthyl-4-[3N-(2-(2-furylméthylamino)-3,4-dioxocyclobutène-1-yl)amino]phényl-1,2,3,4-tétrahydro-isoquinoléine,
la 6,8-dichloro-2-méthyl-4-[3N-(2-cyclopentylamino-3,4-dioxocyclobutène-1-yl)amino]phényl-1,2,3,4-tétrahydro-isoquinoléine,
la 6,8-dichloro-2-méthyl-4-[3N-(2-isopropylamino)-3,4-dioxocyclobutène-1-yl)amino]phényl-1,2,3,4-tétrahydro-isoquinoléine,
la 6,8-dichloro-2-méthyl-4-[3N-(2N-diméthylaminoéthylamino)-3,4-dioxocyclobutène-1-yl)amino]phényl-1,2,3,4-tétrahydro-isoquinoléine,
la 6,8-dichloro-2-méthyl-4(R)-[2N-(2-isopropylamino-3,4-dioxocyclobutène-1-yl)amino]phényl-1,2,3,4-tétrahydro-isoquinoléine,
la 6,8-dichloro-2-méthyl-4(S)-[3N-(2-méthylamino-3,4-dioxocyclobutène-1-yl)amino]phényl-1,2,3,4-tétrahydro-isoquinoléine et
la 5,8-dichloro-2-méthyl-4(R)-[2N-(2-méthylamino-3,4-dioxocyclobutène-1-yl)amino]phényl-1,2,3,4-tétrahydro-isoquinoléine
ainsi que leurs sels pharmaceutiquement acceptables et leurs trifluoroacétates.

5. Composés de formule 1 et leurs sels pharmaceutiquement acceptables selon l'une ou plusieurs des revendications 1 à 4 pour une utilisation comme médicament.

6. Utilisation d'un composé de formule 1 et/ou de ses sels pharmaceutiquement acceptables selon l'une ou plusieurs des revendications 1 à 4 pour la préparation d'un médicament destiné au traitement ou à la prophylaxie de troubles de la fonction respiratoire, de troubles respiratoires, de troubles respiratoires provoqués par le sommeil, d'apnées du sommeil, du ronflement, de maladies rénales et chroniques, de l'insuffisance rénale aiguë et chronique, de troubles de la fonction intestinale, de l'hypertension, de l'hypertonie essentielle, de maladies du système nerveux central, de maladies qui résultent d'une surexcitabilité du SNC, de l'épilepsie et de crampes déclenchées par voie centrale ou d'états d'anxiété, de dépressions et de psychoses, d'états ischémiques du système nerveux périphérique ou central ou d'une attaque, de lésinons et maladies aiguës et chroniques d'organes périphériques ou de membres qui sont provoquées par des phénomènes d'ischémie ou de reperfusion, de l'athérosclérose, de troubles du métabolisme, de thromboses, de troubles de la fonction biliaire, d'une attaque par des ectoparasites, de maladies suite à un dysfonctionnement endothélial, de maladies provoquées par des protozoaires, de la malaria, d'états de choc et du diabète et des lésinons tardives diabétiques ou de maladies dans lesquelles la prolifération cellulaire représente une cause primaire ou secondaire, pour la conservation ou l'entreposage de greffes en vue de mesures chirurgicales, pour l'utilisation lors d'opérations chirurgicales et de transplantations d'organes et pour la préservation de la santé et l'allongement de la vie.

7. Utilisation d'un composé de formule I et/ou de ses sels pharmaceutiquement acceptables selon l'une ou plusieurs des revendications 1 à 4 en combinaison avec d'autres médicaments ou substances actives pour la préparation d'un médicament destiné au traitement ou à la prophylaxie de troubles de la fonction respiratoire, de troubles respiratoires, de troubles respiratoires provoqués par le sommeil, d'apnées du sommeil, du ronflement, de maladies rénales et chroniques, de l'insuffisance rénale aiguë et chronique, de troubles de la fonction intestinale, de l'hypertension, de l'hypertonie essentielle, de maladies du système nerveux central, de maladies qui résultent d'une surexcitabilité du SNC, de l'épilepsie et de crampes déclenchées par voie centrale ou d'états d'anxiété, de dépressions et de psychoses, d'états ischémiques du système nerveux périphérique ou central ou d'une attaque, de lésions et maladies aiguës et chroniques d'organes périphériques ou de membres qui sont provoquées par des phénomènes d'ischémie ou de reperfusion, de l'athérosclérose, de troubles du métabolisme, de thromboses, de troubles de la fonction. biliaire, d'une attaque par des ectoparasites, de maladies suite à un dysfonctionnement endothélial, de maladies provoquées par des protozoaires, de la malaria, d'états de choc ou du diabète et des lésions tardives diabétiques ou de maladies dans lesquelles la prolifération cellulaire représente une cause primaire ou secondaire, pour la conservation ou l'entreposage de greffes en vue de mesures chirurgicales, pour l'utilisation lors d'opérations chirurgicales et de transplantations d'organes et pour la préservation de la santé et l'allongement de la vie.

8. Utilisation d'un composé de formule 1 et/ou de ses sels pharmaceutiquement acceptables selon l'une ou plusieurs des revendications 1 à 4, seul(s) ou en combinaison avec d'autres médicaments ou substances actives pour la préparation d'un médicament destiné au traitement ou à la prophylaxie de troubles de la fonction respiratoire et/ou de troubles respiratoires provoqués par le sommeil, tels que les apnées du sommeil.

9. Utilisation d'un composé de formule 1 et/ou de ses sels pharmaceutiquement acceptables selon l'une ou plusieurs des revendications 1 à 4, seul(s) ou en combinaison avec d'autres médicaments ou substances actives pour la préparation d'un médicament destiné au traitement ou à la prophylaxie du ronflement.

10. Utilisation d'un composé de formule 1 et/ou de ses sels pharmaceutiquement acceptables selon l'une ou plusieurs des revendications 1 à 4, seul(s) ou en combinaison avec d'autres médicaments ou substances actives pour la préparation d'un médicament destiné au traitement ou à la prophylaxie de maladies rénales aiguës ou chroniques, de l'insuffisance rénale aiguë ou de l'insuffisance rénale chronique.

11. Utilisation d'un composé de formule I et/ou de ses sels pharmaceutiquement acceptables selon l'une ou plusieurs des revendications 1 à 4, seul(s) ou en combinaison avec d'autres médicaments ou substances actives pour la préparation d'un médicament destiné au traitement ou à la prophylaxie de troubles de la fonction intestinale.

12. Remède destiné à une utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 4.

13. Remède destiné à une utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 4, en combinaison avec une ou plusieurs autres substances actives pharmacologiques ou un ou plusieurs autres médicaments.
